# EUROPEAN PATENT APPLICATION

(11) **EP 4 715 366 A1**
(43) Date of publication of application: **25.03.2026**
(21) Application number: 24807277.9
(22) Date of filing: 16.05.2024
(51) Int. Cl.: G01N 21/01, A61B 5/1455, G01J 3/10, G01N 21/3577, H01K 1/06

(54) **INFRARED ANALYSIS CHIP, WEARABLE DEVICE, AND INFRARED ANALYSIS DEVICE**

(30) Priority: 17.05.2023 JP 2023081419
(71) Applicant: Keio University, Tokyo, 108-8345 (JP)
(72) Inventor: MAKI, Hideyuki, Yokohama-shi, Kanagawa 223-8522 (JP)
(74) Representative: Piotrowicz, Pawel Jan Andrzej
(86) International application number: PCT/JP2024/018235
(87) International publication number: WO 2024/237327

(57) **Abstract**

An infrared analysis chip includes a first substrate, a second substrate facing the first substrate, a flow path provided between the first substrate and the second substrate, and one or more nanocarbon light sources thermally insulated from the flow path and configured to irradiate the flow path with infrared light.

## Description

### TECHNICAL FIELD

The present invention relates to an infrared analysis chip, a wearable device, and an infrared analysis apparatus, and more particularly to a compact infrared analysis chip that enables various analyses with a simple configuration, a wearable device, and an infrared analysis apparatus.

### BACKGROUND ART

Currently, liquid airtight cells are used for transmission measurement by infrared analysis. A liquid airtight cell is a large cell in which a spacer is interposed between two infrared transmission windows using a large jig, and liquid is encapsulated with a gasket. The liquid airtight part is large, several centimeters or more, and the liquid encapsulating portion needs to be fixed by a metal jig or the like with a plate, a screw, or the like, and handling for encapsulating the liquid is complicated and expensive. Therefore, disposable liquid airtight cells have not been realized. Since the liquid encapsulating part is large, a large amount of liquid is needed, making it difficult to use for simple infrared analysis or inspection. Conventional infrared measurement of liquids uses an external macro light source of about a few centimeters in size, such as a halogen lamp or a ceramic light source, equipped with a Fourier transform infrared spectrophotometer (FTIR) to irradiate a sample with light. Since the size of a halogen lamp or a ceramic light source is large, a large instrument or system, such as microspectroscopy, is separately needed to measure a small amount of a sample or to measure the position dependence of a sample. In the case where microspectroscopy or large cells with liquid encapsulation is used, infrared analysis with fluids or other liquids flowing through the system is not easily performed. To perform spectroscopy, a spectroscopic system, such as a Michelson interference system or a diffraction grating, is essential.

A light source using graphene (for example, see Patent Document 1) and a light-emitting device using carbon nanotubes (CNT) (for example, see Patent Document 2) are known. Infrared analysis and infrared imaging technologies using a light source made of nanocarbon materials, such as graphene and CNT, have been proposed (for example, see Patent Document 3).

If a compact infrared analysis chip capable of performing infrared analysis and measurement with ease becomes available, it will significantly broaden applications in medical, biological, and environmental fields. When measuring a fluid sample using a minute light source based on nanocarbon materials, a novel control technology is required - one that enables precise fluid manipulation corresponding to the scale of the light source, while keeping a simple flow path configuration.

### RELATED ART DOCUMENTS

### PATENT DOCUMENT

Patent Document 1: Japanese Patent No. 6155012
Patent Document 2: Japanese Patent No. 5747334
Patent Document 3: International Publication No. WO 2019/176705

### SUMMARY OF THE INVENTION

### PROBLEMS TO BE SOLVED BY THE INVENTION

It is an object of the present invention to provide a compact infrared analysis chip that enables various analyses and measurements with a simple configuration, a wearable device, and an infrared analysis apparatus.

### MEANS FOR SOLVING THE PROBLEMS

In one aspect of the present invention, an infrared analysis chip includes: a first substrate; a second substrate facing the first substrate; a flow path provided between the first substrate and the second substrate; and one or more nanocarbon light sources thermally insulated from the flow path and configured to irradiate the flow path with infrared light.

In another aspect of the present invention, an infrared analysis chip includes: a first substrate; a second substrate facing the first substrate; a spacer separating the first substrate and the second substrate at a predetermined interval; and a flow path formed by the spacer between the first substrate and the second substrate, wherein at least one of the first substrate or the second substrate is transparent to light of an infrared range, and the spacer has a structure in which an adhesive layer and a spacer member contained in the adhesive layer are integrated.

In yet another aspect of the present invention, an infrared analysis apparatus includes: a substrate; and a nanocarbon light source provided on the substrate and configured to irradiate an object to be measured with infrared light by black body radiation, wherein the nanocarbon light source emits the infrared light modulated in such a manner that a second temperature difference becomes 0.2 times or less of a first temperature difference, the first temperature difference being a temperature difference in the substrate between when a voltage or current of a first value is continuously applied to the nanocarbon light source and when a voltage or current of a second value is continuously applied to the nanocarbon light source, the second temperature difference being a temperature difference between a maximum temperature of the substrate and a minimum temperature of the substrate when the voltage or the current is modulated in such a manner that the voltage or current of the first value is applied as a maximum value and the voltage or current of the second value is applied as a minimum value.

In yet another aspect of the present invention, an infrared analysis chip includes:
a flow path into which liquid is introduced; and a nanocarbon heater provided in the flow path, wherein the nanocarbon heater is provided in the flow path and configured to locally heat the liquid by applying a voltage to generate bubbles, and the bubbles cause the liquid to move within the flow path.

In yet another aspect of the present invention, a wearable device having an infrared analysis chip includes: a first substrate having a surface configured to be in contact with a living body; a third substrate configured to support the first substrate; and a nanocarbon light source provided between the first substrate and the third substrate and configured to irradiate the living body with infrared light, wherein the first substrate is formed of a material having a refractive index higher than that of the living body, and reflected infrared light reflected by the living body or scattered infrared light scattered by the living body is detected on a back side of the third substrate.

In yet another aspect of the present invention, an infrared analysis chip includes: a flow path; a plurality of nanocarbon light sources configured to irradiate the flow path with infrared light; and a light-receiving element configured to receive infrared light transmitted through, reflected by, or scattered by the flow path, wherein the plurality of nanocarbon light sources are different in size, emission intensity of the infrared light, wavelength of the infrared light, modulation rate of the infrared light, modulation frequency of the infrared light, or emission timing.

### EFFECTS OF THE INVENTION

Provided is an infrared analysis chip which can perform various infrared analyses and measurements with a simple configuration.

### BRIEF DESCRIPTION OF THE DRAWINGS

[FIG. 1A] FIG. 1A is a schematic top view of an infrared analysis chip according to a first embodiment.
[FIG. 1B] FIG. 1B is a cross-sectional view taken along line I-I of FIG. 1A.
[FIG. 2A] FIG. 2A is a schematic top view of a first modified example of the infrared analysis chip according to the first embodiment.
[FIG. 2B] FIG. 2B is a cross-sectional view taken along line II-II of FIG. 2A.
[FIG. 3A] FIG. 3A is a schematic top view of a second modified example of the infrared analysis chip according to the first embodiment.
[FIG. 3B] FIG. 3B is a cross-sectional view taken along line III-III of FIG. 3A.
[FIG. 4] FIG. 4 is a schematic top view of a third modified example of the infrared analysis chip according to the first embodiment.
[FIG. 5] FIG. 5 illustrates an example of a use of the infrared analysis chip of the first embodiment.
[FIG. 6A] FIG. 6A is a schematic top view of an infrared analysis chip according to a second embodiment.
[FIG. 6B] FIG. 6B is a cross-sectional view taken along line VI-VI of FIG. 6A.
[FIG. 7A] FIG. 7A a diagram illustrating extraction of modulated light from a nanocarbon light source.
[FIG. 7B] FIG. 7B is a schematic diagram illustrating substrate temperature with respect to time.
[FIG. 8] FIG. 8 is a schematic cross-sectional view of a first modified example of the infrared analysis chip according to the second embodiment.
[FIG. 9] FIG. 9 is a schematic cross-sectional view of a second modified example of the infrared analysis chip according to the second embodiment.
[FIG. 10A] FIG. 10A is a schematic top view of an infrared analysis chip according to a third embodiment.
[FIG. 10B] FIG. 10B is a cross-sectional view taken along line IX-IX of FIG. 10A.
[FIG. 11A] FIG. 11A is a schematic top view of a modified example of the infrared analysis chip according to the third embodiment.
[FIG. 11B] FIG. 11B is a cross-sectional view taken along line X-X of FIG. 11A.
[FIG. 12] FIG. 12 is a schematic top view of an infrared analysis chip according to a fourth embodiment.
[FIG. 13A] FIG. 13A is a diagram illustrating an example configuration in a cross section taken along line XI-XI of FIG. 12.
[FIG. 13B] FIG. 13B is a diagram illustrating an example configuration in a cross section taken along line XI-XI of FIG. 12.
[FIG. 14] FIG. 14 is a schematic perspective view of an infrared analysis chip according to a fifth embodiment.
[FIG. 15] FIG. 15 is a diagram illustrating a cross-sectional configuration example of the infrared analysis chip of FIG. 14.
[FIG. 16A] FIG. 16A is a schematic cross-sectional view of an infrared analysis chip according to a sixth embodiment.
[FIG. 16B] FIG. 16B is a schematic cross-sectional view of the infrared analysis chip according to the sixth embodiment.
[FIG. 17A] FIG. 17A is a diagram illustrating a modified example of the infrared analysis chip according to the sixth embodiment of FIGS. 16A and 16B.
[FIG. 17B] FIG. 17B is a diagram illustrating another modified example of the infrared analysis chip according to the sixth embodiment of FIGS. 16A and 16B.
[FIG. 17C] FIG. 17C is a diagram illustrating yet another modified example of the infrared analysis chip according to the sixth embodiment of FIGS. 16A and 16B.
[FIG. 18] FIG. 18 is a schematic cross-sectional view of an infrared analysis chip according to a seventh embodiment.
[FIG. 19] FIG. 19 is a schematic diagram illustrating an arrangement example of the infrared analysis chip of FIG. 18.
[FIG. 20] FIG. 20 is a schematic diagram illustrating another arrangement example of the infrared analysis chip of FIG. 18.
[FIG. 21] FIG. 21 is an image of an infrared analysis chip of the first embodiment actually manufactured.
[FIG. 22] FIG. 22 is an image of an infrared analysis chip of the second embodiment actually manufactured.
[FIG. 23A] FIG. 23A is an image of bubble generation in a flow path of the seventh embodiment.
[FIG. 23B] FIG. 23B is an image of bubble generation in a flow path of the seventh embodiment.
[FIG. 24A] FIG. 24A is a graph showing an infrared absorption spectrum of ethylene glycol measured with the infrared analysis chip of FIGS. 10A and 10B.
[FIG. 24B] FIG. 24B is a graph showing an infrared absorption spectrum of dimethyl sulfoxide measured with the infrared analysis chip of FIGS. 10A and 10B.
[FIG. 25A] FIG. 25A is a diagram illustrating measurement results of glucose using the infrared analysis chip of FIGS. 10A and 10B.
[FIG. 25B] FIG. 25B is a diagram illustrating measurement results of glucose using the infrared analysis chip of FIGS. 10A and 10B.
[FIG. 25C] FIG. 25C is a diagram illustrating measurement results of glucose using the infrared analysis chip of FIGS. 10A and 10B.
[FIG. 26] FIG. 26 is a schematic cross-sectional view of an infrared analysis chip according to an eighth embodiment.
[FIG. 27] FIG. 27 is a schematic cross-sectional view of a modified example of the infrared analysis chip according to the eighth embodiment.
[FIG. 28] FIG. 28 is a schematic cross-sectional view of a wearable device according to a ninth embodiment.
[FIG. 29] FIG. 29 is a top view of a nanocarbon heater and its vicinity in a modified example of the sixth embodiment and a modified example of the seventh embodiment.
[FIG. 30] FIG. 30 is a schematic view of an infrared analysis apparatus according to a tenth embodiment.

### DESCRIPTION OF EMBODIMENTS

Hereinafter, infrared analysis chips of embodiments will be described in detail with reference to the drawings. The embodiments described below are examples for embodying the technical concept of the invention, and the invention is not limited to the following configurations and numerical values. In the drawings, components having the same function may be given the same reference numerals and redundant descriptions may be omitted. Partial replacement or combination between different embodiments and configuration examples is possible. The sizes, positional relationships, and the like of each member illustrated in each drawing may be exaggerated to facilitate understanding of the invention.

In the embodiments, infrared analysis chips which enable various infrared analyses with a simple configuration are provided. The infrared analysis chips can be cost-effectively manufactured with a simple flow path configuration and can be configured to be disposable or reusable. A minute nanocarbon light source or a nanocarbon heater is incorporated in the infrared analysis chips to enable infrared analysis of a minute amount of liquid. Herein, a "nanocarbon light source" is a light source having a nanocarbon material, such as CNT, graphene, multilayer graphene, or fullerene nanofiber, as a light emitter. A plurality of nanocarbon light sources may be incorporated in the infrared analysis chips. In this configuration, various infrared analysis measurements can be performed with one infrared analysis chip by controlling a plurality of nanocarbon light sources individually to vary emission timing, emission intensity, wavelength, modulation frequency, modulation rate, and other emission characteristics. A plurality of nanocarbon devices may be separately used as a nanocarbon light source for infrared light irradiation and a nanocarbon heater for local heating. The nanocarbon heater is a heat source utilizing heat generation by black body radiation of the above-mentioned nanocarbon material. When the nanocarbon device is used as a nanocarbon heater, liquid introduced into the flow path is locally heated to generate bubbles, and the liquid can be propelled or moved in a flow path by pumping using the expansion and contraction of the bubbles.

### <First Embodiment>

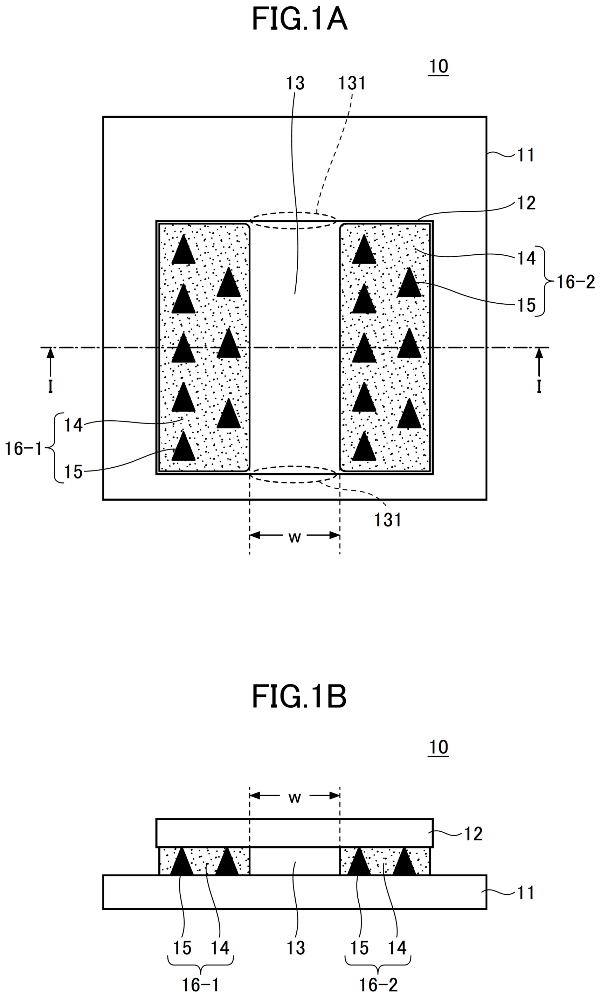

FIG. 1A is a schematic top view of an infrared analysis chip 10 according to the first embodiment, and FIG. 1B is a cross-sectional view taken along line I-I of FIG. 1A. The infrared analysis chip 10 includes a first substrate 11, a second substrate 12 facing the first substrate 11, spacers 16-1 and 16-2 for separating the first substrate 11 and the second substrate 12 at predetermined intervals, and a flow path 13 formed by the spacers 16-1 and 16-2 between the first substrate 11 and the second substrate 12. In the example of FIGS. 1A and 1B, the first substrate 11 refers to the upper substrate and the second substrate 12 refers to the lower substrate for convenience, but either substrate may be referred to as "the first substrate". At least one of the first substrate 11 or the second substrate 12 is transparent to light in the infrared range. Herein, "transparent" to light in the infrared range means having a transmittance of 20% or more, preferably 70% or more, more preferably 80% or more, and even more preferably 90% or more, in a wavelength range targeted for analysis. The infrared range is a mid-infrared range having a wavelength of 1 µm or greater, typically 2 µm or greater, on the short-wavelength side, and a wavelength of 40 µm or less, typically 20 µm or less, on the long-wavelength side. As long as light is transmitted within a wavelength range to be analyzed, substrate materials can include not only inorganic materials but also organic materials such as resins and plastics. It is not a problem if a substrate material absorbs light at wavelengths other than the one targeted for analysis.

Each of the spacers 16-1 and 16-2 has a structure in which an adhesive layer 14 and a spacer member 15 contained in the adhesive layer 14 are integrated. The spacers 16-1 and 16-2 constitute side walls of the flow path 13 between the first substrate 11 and the second substrate 12. In FIGS. 1A and 1B, the flow path 13 extending in one direction is formed by two spacers 16-1 and 16-2 (hereinafter, they may be simply referred to as "spacer 16"), but the number of the spacers 16 and the shape of the flow path 13 are not particularly limited. A single spacer 16 provided along the outer peripheries of the first substrate 11 and the second substrate 12 may form one flow path in the center of the substrate, or two spacers 16 may form a flow path in an L-shape, U-shape, meander shape, annular shape, or other similar configurations, and extending to the end face of the substrate. Three spacers may be arranged to form a Y-shaped flow path, or four spacers may be used to form a cross-shaped or X-shaped flow path extending to the end face of the substrate. To efficiently introduce liquid into the flow path, it is desirable to have openings at two or more locations.

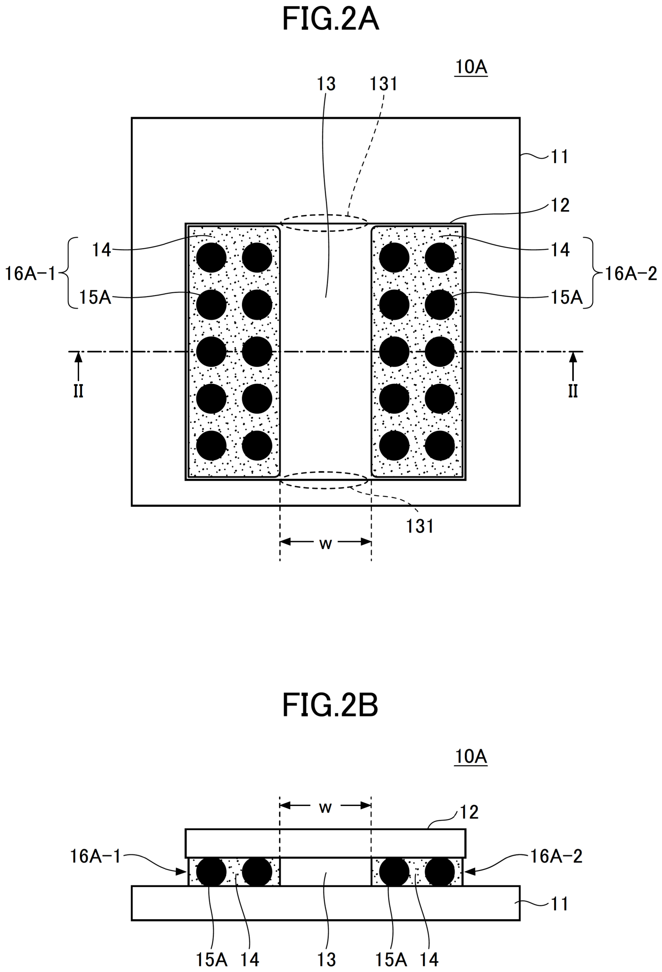

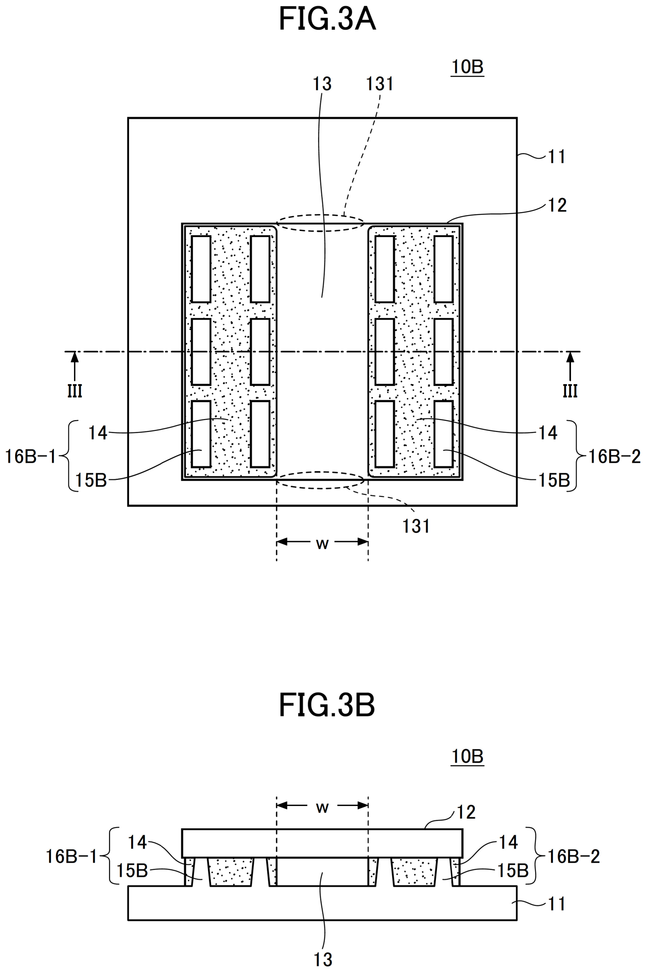

The spacer member 15 may be any member as long as it can support the second substrate 12 on the first substrate 11 before the adhesive layer 14 is cured. As an adhesive, any adhesive may be used, but for example, an adhesive that is cured by ultraviolet light, an adhesive that is cured by temperature, an adhesive that is cured by mixing a plurality of liquids, an adhesive that is reversibly cured or melted by temperature, or the like, may be used. As the material of the adhesive layer 14, either a resin-based adhesive or an inorganic-based adhesive may be used. As will be described later with reference to FIGS. 2A, 2B, 3A, and 3B, the spacer member 15 may consist of projections formed on the first substrate 11 or the second substrate 12 by a lithographic technique or may consist of particles. The particles may be, for example, microbeads, glass flakes, hollow glass particles, a low dielectric constant filler, or other filler. The material of the particles may be plastic, glass, or other material. Preferably, the particles are fine particles (particles in a specific shape) having a specific diameter and a specific shape and having substantially the same size and shape. The shape may be spherical or may be other than spherical. The size (diameter) is preferably 1 µm to 1 mm, for example. Generally, the size is 5 µm to 500 µm, and 5 µm, 10 µm, 50 µm, or 100 µm. In particular, in the case of using the particles by mixing them with an adhesive, the process is simplified, as it only requires mixing the particles with the adhesive. This enables low-cost production of the infrared analysis chip and allows easy formation of a liquid layer with a desired thickness. Gaskets, spacer sheets, screws, and jigs for fixing them are unnecessary, and since the infrared analysis chip is compact in size, it can be easily mounted on various analysis apparatuses. Since the infrared analysis chip is low in cost, it is disposable, and since it does not have screws, etc., it can be cleaned as it is without requiring disassembly. The distance between the first substrate 11 and the second substrate 12, that is, the height of the flow path 13, depends on the type, size, and hardness of the spacer member 15, viscosity of the adhesive layer 14, and the like, but is, for example, 1 µm or greater and 10 mm or less, preferably 1 mm or less, and more preferably 500 µm or less. Typically, a width of 10 µm or greater and 100 µm or less is used. The width w of the flow path 13 is, for example, 1 µm or greater, typically 10 µm or greater. The upper limit of the width w does not need to be set, but is typically 30 mm or less, and is often 1 mm or less.

Liquid to be measured is introduced into the flow path 13. Liquid may be introduced into the flow path 13 in any manner. For example, if liquid is dropped into an opening 131 at the end of the flow path 13, the liquid automatically permeates into the flow path 13 by a capillary phenomenon. It is not necessary to use a capillary phenomenon, and liquid may be introduced into the flow path 13 and discharged from the flow path 13 by injection and suction using a manipulator (e.g., a syringe, a micropump, an injector, an aspirator, or the like), as described later. In the case of a volatile liquid sample, the opening 131 of the flow path 13 may be sealed with an adhesive, a photocurable resin, or the like after being filled with the liquid. The sizes of the first substrate 11 and the second substrate 12 may be the same or different. In the case where the sizes of the first substrate 11 and the second substrate 12 are the same, the liquid may be filled into the flow path 13 by dropping the liquid from an end face of the substrate into the opening 131 of the flow path 13, or by dipping an edge portion of the substrate including the opening 131 of the flow path 13 into the liquid. As described above, the liquid may be introduced into the flow path 13 by applying pressure or suction.

The first substrate 11 or the second substrate 12 may be any substrate as long as it can transmit infrared light used for measurement. For example, CaF₂, BaF₂, Ge, Si, KBr, KSR-5, NaCl, ZnSe, ZnS, CsI, SiO₂, Al₂O₃, or the like may be used as a material. These substrates have transmittance of 20% or more, preferably 70% or more, more preferably 80% or more, and even more preferably 90% or more in the wavelength range used for analysis in the infrared range. Depending on an object to be measured, a substrate having a high transmittance especially for mid-infrared light may be used. As long as light is transmitted within the wavelength range of infrared light to be analyzed, substrate materials include not only inorganic materials but also organic materials, such as resins and plastics.

The infrared analysis chip 10 can be manufactured, for example, by the following procedure: (a) the first substrate 11 and the second substrate 12 at least one of which is transparent to infrared light are prepared; (b) the adhesive layer 14 containing the spacer member 15 is arranged between the first substrate 11 and the second substrate 12; (c) the first substrate 11 and the second substrate 12 are fixed at a predetermined interval by the spacer 16 in which the adhesive layer 14 and the spacer member 15 are integrated; and (d) the flow path 13 is formed by the spacer 16 between the first substrate 11 and the second substrate 12. Unlike conventional liquid cells for infrared analysis in which a sheet-shaped spacer and a gasket are fixed and sealed between substrates with screws, the flow path 13 having a desired width, length, and height can be formed only by applying and curing the adhesive layer 14 containing the spacer member 15 at a desired position on the substrate. Since fixation with a gasket or screw is not used, a compact analysis chip can be mass-produced cost-effectively. Therefore, a disposable analysis chip can be made, and since there are no screws or comparable fastening components, the analysis chip can be cleaned without requiring disassembly.

By using the space formed by the spacer 16 between the first substrate 11 and the second substrate 12 as the flow path 13 and introducing a liquid sample into this flow path, a very thin liquid layer is formed inside the infrared analysis chip 10. The thickness of the liquid layer can be freely designed from the order of 1 µm to the order of 1 mm by changing the height of the spacer 16. The width w of the flow path 13 can be freely designed in accordance with the sizes of the first substrate 11 and the second substrate 12 and the application area of the adhesive layer 14, and typically can be freely designed from the order of 10 µm to the order of several centimeters. The shape of the flow path 13 can also be freely designed following the shape of the application area of the adhesive layer 14. The width w may be varied along the flow path 13. For example, the width w may be widened or narrowed toward the opening 131 of the flow path 13. By narrowing the width of the opening of the flow path 13, volatilization of liquid can be suppressed. In conventional liquid cells for infrared analysis, the width of the flow path cannot be changed by design because the gasket and the spacer sheet are fixed by screws. However, the infrared analysis chip according to the present embodiment is characterized in that the width of the flow path can be changed simply by changing the application area of the adhesive. The infrared analysis chip according to the present embodiment is furthermore characterized in that the thickness of the liquid layer can be easily changed simply by mixing the spacer member, such as projections or particles, with an adhesive. Therefore, a compact, cost-effective, and disposable, infrared analysis chip can be made. In particular, the infrared analysis chip is characterized in that it can be easily mounted on various types of analysis equipment because of its compact size.

The infrared analysis chip 10 is disposable and reusable. After a liquid sample is injected into the flow path 13 and infrared measurement is performed, the liquid sample is suctioned out using a suction machine with a hose or the like, and the inside of the flow path 13 can be cleaned by repeating the injection and suction of pure water into the flow path 13. The infrared analysis chip 10 is useful not only as a disposable chip but also as a reusable chip that can be easily manufactured at a low cost.

FIG. 2A is a schematic top view of an infrared analysis chip 10A of a first modified example of the first embodiment, and FIG. 2B is a sectional view taken along line II-II of FIG. 2A. In the first modified example, particles 15A are used as a spacer member contained in spacers 16A-1 and spacers 16A-2 (may be collectively referred to as "spacer 16A" hereinafter). A space for the flow path 13 is secured between the first substrate 11 and the second substrate 12 by the particles 15A contained in the adhesive layer 14. The adhesive layer 14 in which the particles 15A are dispersed is applied to a necessary part of the first substrate 11 or the second substrate 12 and cured, whereby the particles 15A and the adhesive layer 14 are integrated to form the spacer 16A.

The infrared analysis chip 10A does not require conventionally needed sheet-like spacers and gaskets and is manufactured cost-effectively with a simple structure. In FIGS. 2A and 2B, the particles 15A contained in the spacer 16A are depicted as being regularly arranged, but the particles 15A dispersed in the adhesive layer 14 may be randomly arranged. The formation of the flow path 13 of a desired shape between the first substrate 11 and the second substrate 12 by two or more spacers 16A is similar to that of the infrared analysis chip 10 of FIGS. 1A and 1B. A liquid sample is introduced from the opening 131 of the flow path 13.

FIG. 3A is a schematic top view of an infrared analysis chip 10B of the second modified example of the first embodiment, and FIG. 3B is a sectional view taken along line III-III of FIG. 3A. In the second modified example, projections 15B formed on the first substrate 11 or the second substrate 12 are used as a spacer member included in the spacers 16B-1 and 16B-2 (may be collectively referred to as "spacer 16B" hereinafter). The projections 15B are formed in a desired height and shape on the surface of the first substrate 11 or the second substrate 12 by an ordinary lithography technique or etching technique. A space for the flow path 13 is secured between the first substrate 11 and the second substrate 12 by the projections 15B. The adhesive layer 14 is filled between the plurality of projections 15B, the first substrate 11 and the second substrate 12 are overlaid, and the adhesive layer 14 is cured, whereby the projections 15B and the adhesive layer 14 are integrated to form the spacer 16B. The infrared analysis chip 10B does not require conventionally needed sheet-like spacers and gaskets and is manufactured cost-effectively with a simple structure. Although a plurality of island-shaped projections 15B are formed in the configuration examples of FIGS. 3A and 3B, columnar projections such as cylinders, square prisms, and hexagonal prisms may be formed at predetermined intervals or at random, or continuous projections in the shape of walls may be formed. A resist used in lithography technology may be used as projections after lithography. In this method, processing accuracy of a lateral shape as required for an ordinary semiconductor is not required, and any processing method or shape may be used as long as a height capable of obtaining the desired thickness of a liquid layer is obtained. For example, there is no issue with using a projection structure formed by wet etching in a liquid phase, even if the processing accuracy is low. Therefore, the projection structure can be formed using a significantly cost-effective processing process and can be used as a disposable analysis chip.

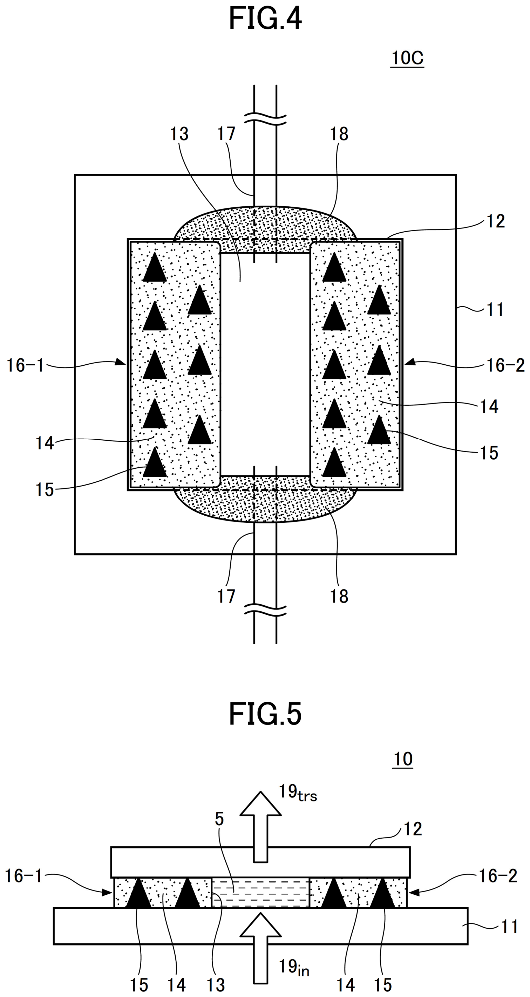

FIG. 4 is a schematic top view of a third modified example of an infrared analysis chip 10C according to the first embodiment. The infrared analysis chip 10C has a sealing member 18 for sealing the opening 131 (see FIGS. 1A through 3B) of the flow path 13 formed by the spacers 16-1 and 16-2. The sealing member 18 is, for example, an ultraviolet curing resin. The material of the sealing member 18 may be the same as or different from the material of the adhesive layer 14 contained in the spacer 16. A tube 17 that penetrates the sealing member 18 and communicates with the internal space of the flow path 13 is provided. The tube 17 is inserted into the flow path 13, and the material of the sealing member 18 is applied to the opening 131 and cured, thereby sealing the flow path 13 and fixing the tube 17.

Although the outer diameter of the tube 17 is 1 µm to several hundred µm in the case where the outer diameter is adjusted to the height of the flow path 13, the outer diameter of the tube is not necessarily adjusted to the height of the flow path, and a tube having an outer diameter greater than the height of the flow path can function by sealing with the sealing material. Therefore, the thickness of the tube can be freely selected, and for example, a tube having an outer diameter of about 50 µm to 10 mm can be used. Typically, a tube having an outer diameter of about 100 µm to 3 mm is easy to handle. Since the thickness of the tube can be freely selected, the tube can be manufactured cost-effectively, and since machining or screwing for fixing the tube is not required, the tube can be manufactured cost-effectively and can be made very small. The tube 17 may be configured to be connectable to an external device, such as a syringe, a microinjector, a micropump, an aspirator, or the like. By connecting a manipulator, such as a syringe, a microinjector, an aspirator, or the like to the tip of the tube 17, liquid can be injected or suctioned into the space of the flow path 13. After liquid is injected, the injection may be terminated and the liquid may be subjected to infrared analysis, or the fluid flowing through the flow path 13 may be subjected to infrared analysis while the injection of the liquid continues. In conventional analysis chips having a flow path, when a tube is connected, a hole is made in either or both of the upper and lower substrates, and a very thick tube is connected to the hole. In FIG. 4, however, drilling of the substrates for connecting a tube is unnecessary, making the chip more compact and cost-effective. This is possible because, as illustrated in FIGS. 1A through 4, the end faces of the two substrates are exposed and opened at least on one side face of the substrate, and introduction of fluid by drilling a hole from above or below the substrate is unnecessary, unlike conventional analysis chips. One or more tubes may be connected by making holes in either or both of the upper and lower substrates. In this case, the side faces of both ends of the substrates may be left open, or either or both sides may be closed with an adhesive or the like. In the case where one or both side faces are closed, liquid can be easily injected or suctioned from the tube. It is also possible to connect two tubes and keep fluid flowing.

The infrared analysis chip 10C is also reusable. After injecting a liquid sample into the flow path 13 and performing infrared measurement, the liquid sample is suctioned, and pure water is injected into and suctioned from the flow path 13 to clean the inside of the flow path 13. The infrared analysis chip 10C is useful as a reusable chip that can be easily manufactured at a low cost.

FIG. 5 illustrates an example of the use of the infrared analysis chip 10 of the first embodiment. Liquid 5 is introduced into the flow path 13, and the flow path 13 is irradiated with infrared light. In the example of FIG. 5, infrared light is incident from the back side of the first substrate 11. The incident light 19in passes through the first substrate 11 and acts on the liquid 5 in the flow path 13, and absorption occurs at a wavelength corresponding to a substance contained in the liquid 5. The light passing through the liquid 5 passes through the second substrate 12. By detecting and measuring the transmitted light 19trs, an infrared absorption spectrum of the liquid 5 is obtained. In the case of measuring transmission through the upper and lower substrates, a material that allows the necessary infrared light for analysis to pass through is selected for both substrates.

In the example of FIG. 5, the infrared absorption spectrum is obtained by transmission measurement, but the infrared analysis chip 10 of the present embodiment is also applicable to measurement with reflected infrared light and scattered infrared light. Similarly, it is also possible to perform liquid measurement with the infrared analysis chips 10A, 10B, and 10C using infrared light transmission, reflection, or scattering. Throughout this specification, analysis using an infrared analysis chip includes infrared analysis using transmitted, reflected, or scattered light. According to the infrared analysis chips 10 and 10A through 10C of the first embodiment, an ultra-compact infrared analysis chip capable of measuring a small amount of liquid is manufactured in a simple manner. When reflection or scattering is used, at least one of the upper and lower substrates is made of a material that allows the necessary infrared light for analysis to pass through.

### <Second Embodiment>

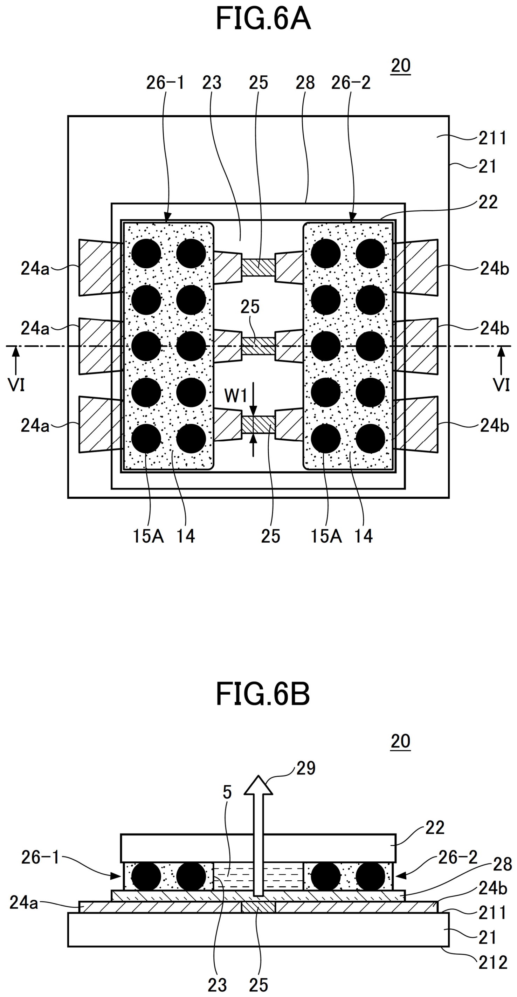

FIG. 6A is a schematic top view of an infrared analysis chip 20 according to the second embodiment, and FIG. 6B is a cross-sectional view taken along line VI-VI of FIG. 6A. In the second embodiment, a nanocarbon light source is mounted on the infrared analysis chip 20. As described above, a "nanocarbon light source" is a light source whose light-emitting part is composed of a nanocarbon material, such as carbon nanotubes (CNT), graphene, multilayer graphene, polycrystalline graphene, fullerenes, and carbon nanofibers. This nanocarbon light source uses a thermal light source based on blackbody radiation induced by Joule heating through energization of the nanocarbon light emitter. By using the infrared analysis chip 20 as an analysis chip with a nanocarbon light source, a thermal light source such as an external halogen lamp or an external infrared light source such as laser light source is not required, and infrared measurement can be performed more simply. The mounted nanocarbon light source may be a single nanocarbon light source or a plurality of nanocarbon light sources.

In the following configuration examples, the flow path structure of the first embodiment, which is simple and small in structure and can have a flow path of a freely chosen shape, is adopted, but the spacer structure of the first embodiment is not required in the second and subsequent embodiments, and a flow path of any configuration may be used.

The infrared analysis chip 20 has a flow path 23 formed by spacers 26-1 and 26-2 (may be collectively referred to as "spacer 26" hereinafter) between the first substrate 21 and the second substrate 22. As described above, the shape of the flow path 23 and the number of the spacers 26 are not particularly limited. The nanocarbon light source 25 for emitting infrared light is provided at a position where the flow path 23 can be irradiated. In this example, the spacer 26 has a configuration in which the particles 15A and the adhesive layer 14 are integrated, but any of the spacers 16, 16A, and 16B of the first embodiment may be used. Instead of a chip having the flow path 23 formed of the spacer 26, a chip having the flow path directly etched into the substrate or a chip sealed with a sealing ring or the like may be used. In the case of using the spacer 26, a meander-shaped flow path 23 may be formed by two spacers 26, and a plurality of nanocarbon light sources 25 may be arranged along the flow path 23. Three or more spacers 26 may be arranged in parallel to form two or more flow paths 23, and a one- or two-dimensional light source array may be constituted by a plurality of nanocarbon light sources 25.

The nanocarbon light source 25 is provided on the surface 211 of the first substrate 21 facing the second substrate 22 or on a back surface 212 to irradiate the flow path 23. When infrared light is irradiated from the upper side to the lower side of the infrared analysis chip 20, the nanocarbon light source 25 may be provided on the second substrate 22. Not only a single nanocarbon light source 25 but also a plurality of nanocarbon light sources 25 may be arranged in an array as illustrated in FIG. 6A. Electrodes 24a and 24b are connected to each of the nanocarbon light sources 25 arranged in an array so that the individual nanocarbon light sources 25 can be driven individually. By applying a voltage between the electrodes 24a and 24b, the nanocarbon material of the nanocarbon light source 25 is energized, and blackbody radiation light emission due to Joule heating is obtained. By varying the emission characteristics of the plurality of nanocarbon light sources 25, such as size, emission timing, emission intensity, emission wavelength, modulation rate, and modulation frequency, various infrared analyses can be performed by a single infrared analysis chip 20.

As illustrated in FIG. 6B, when the nanocarbon light source 25 is arranged on the surface 211 of the first substrate 21 facing the second substrate 22, the first substrate 21 need not be transparent to infrared light, and any kind of substrate can be used. For example, since a cost-effective substrate having excellent mechanical strength can be used, the infrared analysis chip 20 can be manufactured at a lower cost. In the case of using a semiconductor or conductive substrate, the surface of the substrate is covered with an electric insulating film before the nanocarbon light source 25 is formed. In the configuration example of FIG. 6B, infrared light, emitted from the nanocarbon light source 25 by blackbody radiation, acts on the liquid 5 flowing in the flow path 23, and transmitted light 29, which is resulting light of infrared absorption by the liquid's molecules, reaches the side of the second substrate 22. Infrared spectroscopic measurement can be performed by spectrally analyzing the transmitted light 29 by a Michelson interference system or a diffraction grating. In place of transmitted light, scattered infrared light or reflected infrared light can also be used. In the case where reflected light or scattered light is used, the first substrate 21 should be transparent to infrared light in the wavelength range used for analysis.

In the case where the nanocarbon light source 25 is arranged on the surface 211 of the first substrate 21, it is desirable to provide a thermal insulating layer 28 between the flow path 23 and the nanocarbon light source 25. When the liquid 5 introduced into the flow path 23 comes into contact with the nanocarbon light source 25, the liquid 5 may be heated, so the thermal insulating layer 28 is provided between the flow path 23 and the nanocarbon light source 25 to prevent the liquid 5 from heating or boiling. By providing the thermal insulating layer 28, heat escapes to the side of the first substrate 21, and heating of the liquid 5 is suppressed. In FIGS. 6A and 6B, the thermal insulating layer 28 covers the nanocarbon light source 25 and the electrodes 24a and 24b and is formed over a wide area corresponding to the whole of the second substrate 22, but the thermal insulating layer 28 may be locally provided only just above the nanocarbon light source 25 or only in the area facing the flow path 23. By the thermal insulating layer capping the light source, the thermal insulating layer can prevent the nanocarbon light source from being damaged by corrosion, chemical reaction, or the like due to contact between liquid and the light source.

The nanocarbon light source 25 can be continuously used in a steady-state emission mode by applying a direct current (DC) voltage or current, making it suitable for infrared analysis. Furthermore, since the nanocarbon light source 25 can emit light by on-off modulation at a rate of 20 Hz or greater, high-sensitivity infrared analysis can be performed by using a lock-in amplifier or a gate synchronization technique in synchronization with the modulated light emission. The frequency to be modulated may be any modulation frequency that can be realized as long as it is 20 Hz or greater, but typically a frequency of 100 Hz or greater, 500 Hz or greater, 1 kHz or greater, or 10 kHz or greater is used, and a modulation rate of 100 kHz or greater may be used. Although ultrafast lighting up to a maximum of 1 GHz is possible, modulation is typically performed at a maximum of 1 MHz or less or 10 MHz or less in many cases.

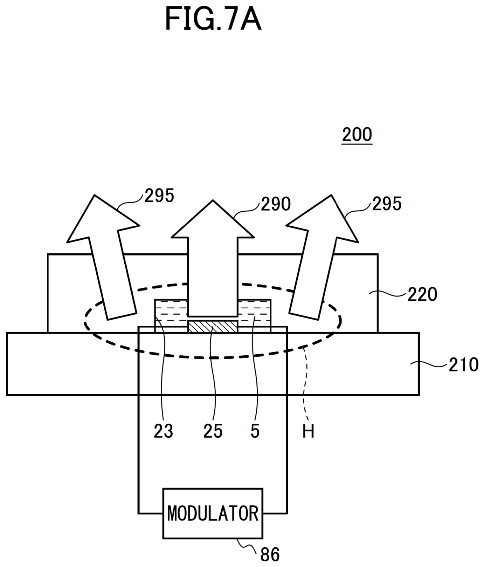

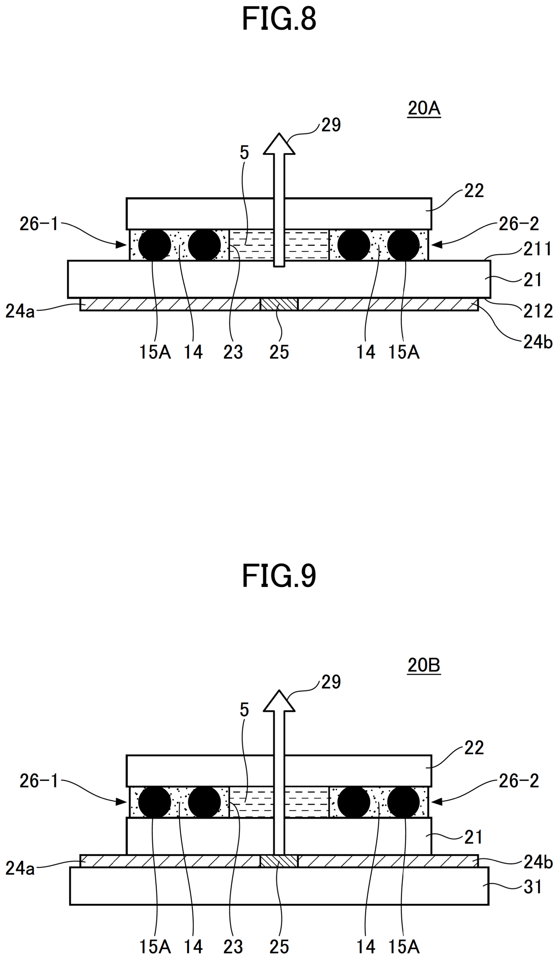

FIG. 7A is a diagram illustrating extraction of modulated light 290 from the nanocarbon light source 25. In an infrared analysis chip 200, the flow path 23 is formed between a first substrate 210 and a second substrate 220, and the nanocarbon light source 25 for irradiating infrared light is arranged in the flow path 23. The nanocarbon light source 25 emits modulated light 290 (modulated infrared light) by controlling an applied pulse. A modulator 86 modulates a voltage or a current applied to the nanocarbon light source 25, thereby causing the nanocarbon light source 25 to emit the modulated light 290. In the analysis by the modulated light source 290, not only high sensitivity can be achieved by measurement in an electric circuit synchronized with light emission, but also only infrared light generated from the nanocarbon light source 25 itself can be selectively received by performing light detection only by a signal synchronized with the modulated light 290. Taking advantage of the thermal stability of the substrates and the liquid sample surrounding the nanocarbon light source 25, it is possible to suppress background radiation of infrared light 295 originating from the first substrate 210, second substrate 220, or liquid 5. Consequently, only the modulated light 290 emitted from the nanocarbon light source 25 can be selectively detected. Thus, infrared analysis using only the modulated light 290 emitted from the nanocarbon light source 25 can be performed, and high-sensitivity infrared analysis can be achieved. In FIG. 7A, as an example, the nanocarbon light source 25 is formed in the flow path 23, but the same effect can be obtained regardless of the position of the light source, for example, even if the light source is located outside the flow path 23 as illustrated in FIGS. 8 and 9, which will be described later. Furthermore, this method can be used not only for an infrared analysis chip but also for an infrared analysis apparatus using a nanocarbon light source in general.

In particular, in a configuration in which the nanocarbon light source 25 is formed on a substrate, the substrate temperature increases and the substrate may generate infrared light, but by blinking the nanocarbon light source 25 at a rate higher than the rate of temperature change of the substrate, only the modulated light 290 from the nanocarbon light source 25 can be extracted, and the sensitivity of analysis can be further enhanced. In consideration of the rate of temperature change of the substrate, the modulation rate of the nanocarbon light source 25 is set to a rate higher than the rate of temperature change of the substrate. Thus, only light emitted from a nanocarbon material having a strictly minute size can be extracted, and spatial resolution of local analysis can be enhanced. For example, as illustrated in FIG. 7A, in the analysis using the flow path 23, the temperature of not only the nanocarbon material forming the nanocarbon light source 25 but also the temperature of the first substrate 210 or the second substrate 220 increases. Since a temperature increase region H caused by the operation of the nanocarbon light source 25 extends to a predetermined range around the nanocarbon light source 25, infrared light from a range wider than the width of the flow path 23 would be detected. In this case, infrared light from other than the liquid in the flow path 23 would be detected the sensitivity of the analysis of the liquid would decrease. With respect to the above, in the case where the nanocarbon light source 25 is driven at a rate higher than the temperature increase rate of the substrate, since the detection of the temperature increase of the substrate can be eliminated, accurately, only light from the flow path 23 can be detected, and the sensitivity of liquid analysis is greatly enhanced. Furthermore, this method can be used not only for an infrared analysis chip but also for an infrared analysis apparatus using a nanocarbon light source in general, and is applicable to an infrared analysis using a nanocarbon light source in general in which an influence of a temperature increase of a substrate on infrared analysis is eliminated.

By setting the modulation rate of the nanocarbon light source 25 higher than the modulation rate of the substrate temperature, the spatial resolution of analysis can be enhanced. Since the temperature modulation of the substrate is typically about several Hertz, the modulation rate of the nanocarbon light source 25 may be set at 10 Hz or greater, more preferably at 100 Hz or greater. It is effective even at 1 kHz or greater. Although this method for enhancing the spatial resolution has been described in connection with the infrared analysis chip 200 having the flow path 23 as an example, it can be commonly used as a method for improving the spatial resolution of any infrared analysis apparatus using a nanocarbon light source. For example, the infrared analysis apparatus may include a substrate and the nanocarbon light source 25 provided on the substrate for irradiating an object to be measured with infrared light, and the nanocarbon light source 25 may emit modulated light at a modulation rate higher than the temperature change of the substrate. The infrared analysis apparatus can be applied to infrared analysis using a general nanocarbon light source.

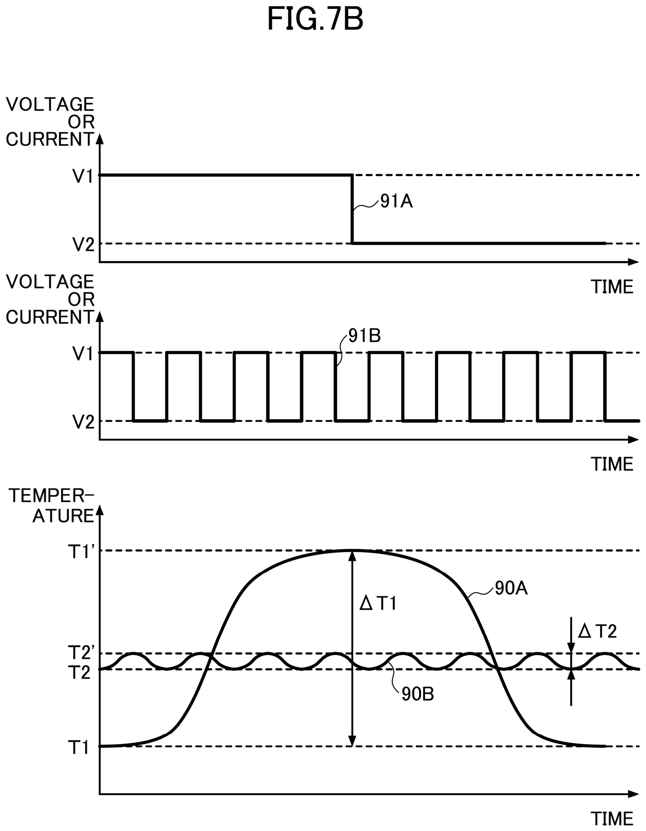

FIG. 7B is a schematic diagram illustrating substrate temperature, voltage, or current with respect to time. In FIG. 7B, the voltage or current on the vertical axis is a voltage or current applied to the nanocarbon light source 25. The temperature on the vertical axis is a temperature of the first substrate 210. The line 91A depicts a voltage or current with respect to time when the voltage or current applied to the nanocarbon light source 25 is modulated at a substantially DC, ultra-low frequency (e.g., less than 1 Hz). The maximum value of the voltage or current is set as a first value V1, and the minimum value of the voltage or current is set as a second value V2. The second value V2 may be 0 V or 0 A. The line 90A depicts a temperature of the first substrate 210 with respect to time when the voltage or current is modulated as depicted by the line 91A. When the voltage or current is modulated at an ultra-low frequency, the temperature of the first substrate 210 changes following the modulation of the voltage or current. Temperature T1 is the minimum temperature of the line 90A and temperature T1' is the maximum temperature of the line 90A. The first temperature difference ΔT1 between temperature T1' and T1 is (T1' - T1). If the frequency of the line 91A is sufficiently low, temperature T1' becomes the temperature when the first value V1 as the voltage or current is continuously applied to the nanocarbon light source 25 and becomes saturated. Temperature T1 becomes the temperature when the second value V2 as the voltage or current is continuously applied to the nanocarbon light source 25 and becomes saturated.

The line 91B depicts the voltage or current with respect to time when the voltage or current applied to the nanocarbon light source 25 is modulated at a frequency higher (e.g., 10 Hz or higher) than that in the case of the line 91A. The maximum value of the voltage or current on the line 91B is the same as the line 91A, i.e., the first value V1, and the minimum value of the voltage or current on the line 91B is the same as the line 91A, i.e., the second value V2. The line 90B depicts the temperature of the first substrate 210 with respect to time when the voltage or current is modulated as depicted by the line 91B. Since the temperature of the first substrate 210 has a slow response rate, minimum temperature T2 of the line 90B increases compared with minimum temperature T1 of the line 90A. Maximum temperature T2' of the line 90B changes only to the extent that the temperature is slightly modulated from maximum temperature T2 of the line 90A. This is because the temperature of the first substrate 210 does not follow the high-rate modulation. The second temperature difference ΔT2 between the temperatures T2' and T2 is (T2' - T2).

The temperature difference ΔT2 is much smaller than the temperature difference ΔT1. The temperature difference ΔT2 is preferably 0.2 times or less of the temperature difference ΔT1, more preferably 0.1 times or less. Although the line 90B is a rectangular wave, the voltage or current applied to the nanocarbon light source 25 can be suitably set, such as a sine wave, a triangle wave, or a sawtooth wave. Although the temperature of the first substrate 210 has been described here, this mechanism can be applied not only to the temperature change of the first substrate 210 but also to the temperature change of any environment surrounding the nanocarbon light source 25 such as the second substrate 220, the flow path 23, and the liquid 5. Furthermore, this method can be used for an infrared analysis apparatus using a nanocarbon light source in general and can be applied to an infrared analysis using a nanocarbon light source in general in which an influence of a temperature increase of a substrate on infrared analysis is eliminated.

FIG. 8 is a schematic cross-sectional view of an infrared analysis chip 20A of the first modified example on which the nanocarbon light source 25 is mounted. In order to avoid contact between liquid 5 in the flow path 23 formed between the spacers 26-1 and 26-2 and the nanocarbon light source 25, the nanocarbon light source 25 is arranged on the surface opposite to the flow path 23, that is, on the back surface 212 of the first substrate 21. The electrodes 24a and 24b connected to the nanocarbon light source 25 are also formed on the back surface 212. In this configuration, the first substrate 21 is transparent to the infrared light emitted from the nanocarbon light source 25 in a wavelength range to be analyzed. The first substrate 21 is thermally insulating and also functions as a thermal insulating layer. In this case, as in FIGS. 6A, 6B, and 7A, by setting the modulation rate of the nanocarbon light source higher than the temperature change rate of the substrate, only the light passing through the flow path is extracted and the infrared light from the substrate not passing through the flow path is excluded from the measurement, thereby enhancing the sensitivity of the measurement.

FIG. 9 is a schematic cross-sectional view of an infrared analysis chip 20B of the second modified example on which the nanocarbon light source 25 is mounted. In FIG. 9, a part including the flow path 23 is prepared by using two infrared light transmitting substrates, and the nanocarbon light source 25 is formed on a third substrate 31. The third substrate 31 on which the nanocarbon light source 25 is formed is joined to the back surface 212 of the first substrate 21 on the side opposite to the surface 211 facing the flow path 23. The electrodes 24a and 24b connected to the nanocarbon light source 25 are also formed on the third substrate 31. In the infrared analysis chip 20B, the nanocarbon light source 25 and the flow path 23 are completely separated by the first substrate 21 being interposed between them, and the problem of heat generated from the nanocarbon light source 25 is sufficiently suppressed. In this case, as in FIGS. 6A, 6B, and 7A, by setting the modulation rate of the nanocarbon light source higher than the temperature change rate of the substrate, only the light passing through the flow path is extracted and the infrared light from the substrate not passing through the flow path is excluded from the measurement, thereby enhancing the sensitivity of the measurement.

Although omitted from FIGS. 6A through 9 for convenience of illustration, a protective film may be attached to the surface of the nanocarbon light source 25 to improve the operation characteristics in the atmosphere and environmental resistance. The protective film may be made of CaF₂, BaF₂, Ge, Si, KBr, KSR-5, NaCl, ZnSe, ZnS, CsI, SiO₂, Al₂O₃ or the like, as long as the protective film is transparent to infrared light in the wavelength range to be analyzed emitted from the nanocarbon light source 25. In addition to the above-mentioned materials having high transmittance in the infrared range, even materials which do not readily transmit infrared light in bulk can transmit infrared light by using the ultra-thin protective film, and therefore any materials, such as inorganic materials, metals and organic materials, which cannot transmit infrared light in bulk can be used as the protective film. Even if the protective film covering the nanocarbon light source 25 is provided, the operation and function of the infrared analysis chips 20, 20A, and 20B are not disturbed. In the structure as illustrated in FIG. 8, for example, when the protective film is provided on the side opposite to the light emission direction of the nanocarbon light source, since the light emitted to the protective film side is not used for analysis, the protective film may be made of a material that does not transmit light at all, and any material and any thickness and shape are acceptable.

In conventional infrared analysis, it is difficult to narrow infrared light to a small size, and even if the beam diameter is narrowed to a minimum by using an expensive objective lens, light can be substantially narrowed to only about several times a wavelength (about several tens of µm). In a microscopic transmission measurement, it is necessary to assemble a complicated and expensive optical system using a plurality of lenses to irradiate and condense light. Therefore, in conventional infrared analysis, it is not easy to narrow infrared light and irradiate the narrow flow path 23 technically and cost-wise. On the other hand, in the second embodiment, the nanocarbon light source 25 is arranged at a position corresponding to the flow path 23 of the infrared analysis chip 20, 20A, or 20B or at a position very adjacent to the flow path, so that the liquid in the minute flow path 23 can be directly irradiated with infrared light. Condensing by an expensive objective lens is therefore unnecessary, and infrared absorption from a liquid sample in the flow path 23 can be obtained cost-effectively and in a simple manner.

In any of the configurations illustrated in FIGS. 6A through 9, an array light source can be constituted by arranging a large number of nanocarbon light sources 25. By using an array light source, infrared analysis spectra at various positions in the flow path 23 can be measured, and infrared analysis measurement depending on the position in the flow path 23 becomes possible. When the presence of particles in the liquid sample to be measured changes depending on the position in the flow path, or the structure and concentration of the substance to be measured changes, it is possible to perform complex infrared analysis using morphology, material structure change, time change, composition or concentration distribution of the particles or substance.

In the case where an array light source is arranged, the timing of emission of the individual nanocarbon light sources 25 can be freely changed at timing of voltage application. Since light emission from the respective nanocarbon light sources 25 can be distinguished and detected by making the plurality of nanocarbon light sources 25 emit light by changing the timing one after another, light emission from the respective nanocarbon light sources 25 can be distinguished and detected by a single detector. In the case where a plurality of nanocarbon light sources 25 are arranged in an array, light sources having the same function and performance may be arranged in an array, or the emission wavelength may be changed by installing a wavelength filter or the like for each nanocarbon light source 25 as described later. In this case, since various wavelengths can be simultaneously analyzed by one element, spectroscopic measurement can be performed without using a spectroscopic device. The size of the nanocarbon light source 25 can be changed for each element of the array by changing the structural design of the light source. Thus, at least one of emission intensity, emission wavelength, modulation rate or modulation frequency, performance of the light source element or the like can be changed for each nanocarbon light source 25. By selecting the nanocarbon light source 25 for irradiating the flow path 23, the nanocarbon light source 25 with the optimum analysis condition can be selected. Even if the nanocarbon light sources 25 have the same characteristics, they may emit light at different frequencies by changing the frequency for driving light emissions. In this case, by performing synchronous measurement according to the frequencies of the respective nanocarbon light sources 25 and simultaneously measuring infrared light of various frequencies, a plurality of light-emitting devices can be distinguished by one detector and simultaneous measurement can be performed.

### <Third Embodiment>

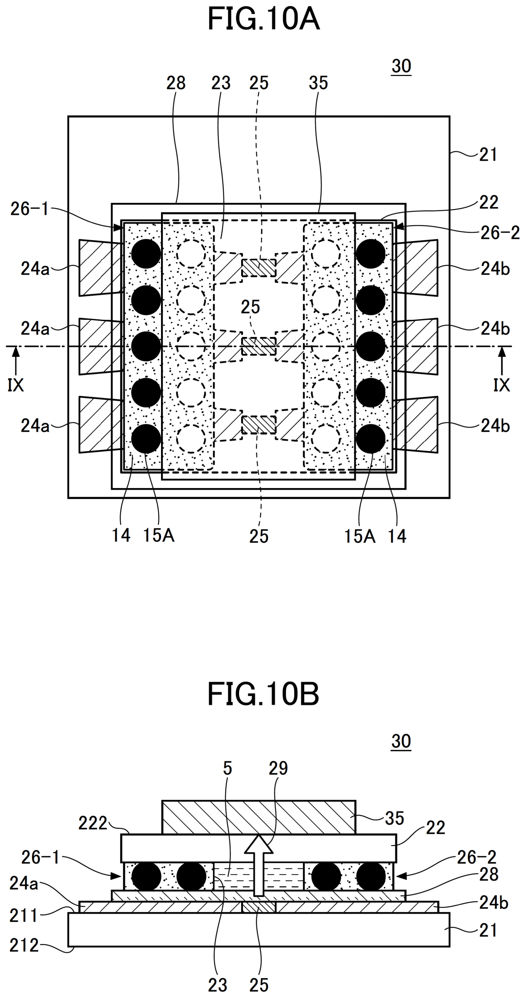

FIG. 10A is a schematic top view of an infrared analysis chip 30 according to the third embodiment, and FIG. 10B is a cross-sectional view taken along line IX-IX of FIG. 10A. In the third embodiment, a light-receiving element 35 is mounted on the infrared analysis chip 30 together with the nanocarbon light source 25. The light-receiving element 35 is sensitive to infrared light emitted from the nanocarbon light source 25. This makes it possible to perform infrared transmission measurements in a complete one-chip configuration. In FIGS. 10A and 10B, the nanocarbon light source 25 is provided on a surface 211 of the first substrate 21 facing the second substrate 22, and the flow path 23 is formed between the spacers 26-1 and 26-2. The thermal insulating layer 28 is provided between the nanocarbon light source 25 and the flow path 23. The light-receiving element 35 is arranged on a top surface 222 of the second substrate 22 (i.e., the surface facing the first substrate 21). The configuration of the infrared analysis chip 30 is not limited to the example of FIGS. 10A and 10B. The nanocarbon light source 25 may be arranged on the back surface 212 of the first substrate to omit the thermal insulating layer 28, and the light-receiving element 35 may be arranged on the top surface 222 of the second substrate 22. In this case, both the first substrate 21 and the second substrate 22 are transparent to the infrared light emitted from the nanocarbon light source 25 in the wavelength range to be analyzed.

One nanocarbon light source 25 may be combined with the light-receiving element 35, or an array of a plurality of nanocarbon light sources 25 may be combined with one light-receiving element 35 as illustrated in FIGS. 6A and 6B. Since the emission of the nanocarbon light sources 25 of an array light source can be individually controlled, the infrared light emitted from the nanocarbon light sources 25 included in an array light source and transmitted through the liquid 5 can be detected by the single light-receiving element 35. Needless to say, the shape of the flow path 23 formed by the spacer 26 between the first substrate 21 and the second substrate 22 can be appropriately designed.

As described above, when a plurality of nanocarbon light sources 25 are arranged in an array, the emission timing of each nanocarbon light source 25 can be freely changed by timing of voltage application. Since light emission from the respective nanocarbon light sources 25 can be distinguished and detected by making the plurality of nanocarbon light sources 25 emit light by changing the timing one after another, light emission from the respective nanocarbon light sources 25 can be distinguished and detected by a single light-receiving element 35. The size of the light source can be changed for each element of the array by changing the structural design of the nanocarbon light sources 25. Thus, at least one of emission intensity, emission wavelength, modulation rate or modulation frequency, or performance of the light source element can be changed for each nanocarbon light source 25. By selecting the nanocarbon light source 25 for irradiating the flow path 23, the nanocarbon light source 25 with the optimum analysis condition can be selected. Even if the nanocarbon light sources 25 have the same characteristics, they may emit light at different frequencies by changing the frequency for driving light emissions. In this case, by performing synchronous measurement according to the frequencies of the respective nanocarbon light sources 25, a plurality of light-emitting devices can be distinguished by one detector and simultaneous measurement can be performed.

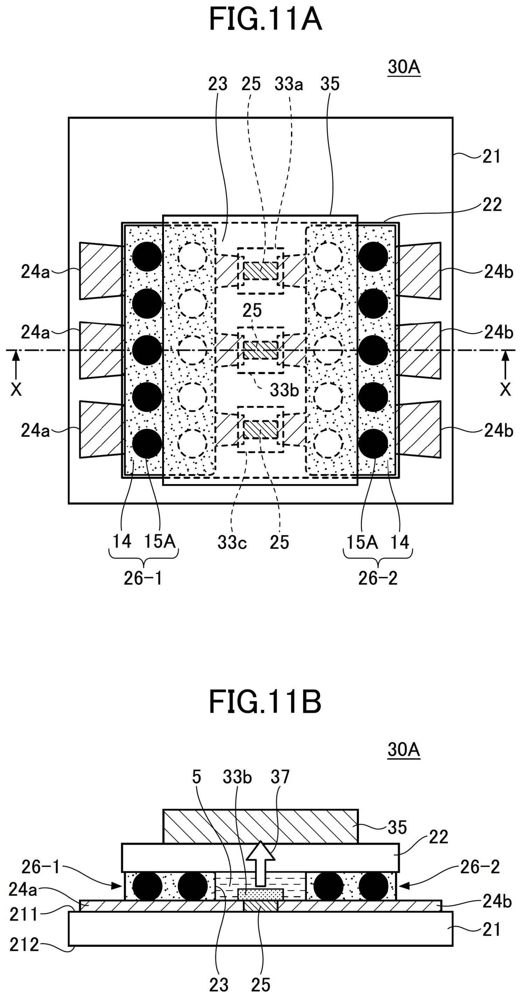

FIG. 11A is a schematic top view of an infrared analysis chip 30A of a modified example on which the light-receiving element 35 is mounted, and FIG. 11B is a cross-sectional view taken along line X-X of FIG. 11A. In the modified example of FIGS. 11A and 11B, the nanocarbon light sources 25 constituting a light source array are provided with filters 33a, 33b, and 33c for passing light of a specific wavelength. The filters 33a, 33b, and 33c (may be collectively referred to as "filter 33" hereinafter) are optical filters, such as a light absorption filter, a dielectric multilayer film filter, and a resonator filter. A multi-wavelength light source array is formed by providing a plurality of nanocarbon light sources 25 with the filters 33a, 33b, and 33c for passing light of different wavelengths. Although it is desirable that each filter 33 is thermally insulated, if it does not have sufficient thermal insulation, the nanocarbon light sources 25 may be covered with a thermally insulated protective film transparent to infrared light, and then the filter 33 may be provided on the protective film. As such a protective film, a thin film of CaF₂, BaF₂, Ge, Si, KBr, KSR-5, NaCl, ZnSe, ZnS, CsI, SiO₂, Al₂O₃, or the like may be used, and any material may be used as long as the thickness is thin, because even if the material in bulk does not transmit infrared light, the thin film allows the infrared light to be transmitted.

By changing the wavelength of infrared light incident on the flow path 23 from each nanocarbon light source 25 with the filter 33, it is possible to measure multiple wavelengths simultaneously. Thus, the multi-wavelength infrared analysis chip 30A for measuring an absorption spectrum without requiring a spectrometer is realized. In the case where a plurality of nanocarbon light sources 25 are arranged in an array, the emission timing of the individual nanocarbon light sources 25 can be freely changed by timing of voltage application. Light emission from the respective nanocarbon light sources 25 can be distinguished and detected by making the plurality of nanocarbon light sources 25 emit light by changing the timing one after another, and light emission from the respective nanocarbon light sources 25 can be distinguished and detected by a single light-receiving element 35. Instead of the filter 33 or together with the filter 33, the respective nanocarbon light sources 25 may be modulated at different frequencies and emitted. In this case, by performing synchronous measurement according to the frequencies of the respective nanocarbon light sources 25 and simultaneously measuring infrared light of various frequencies, the plurality of nanocarbon light sources 25 can be distinguished by one detector and simultaneous infrared spectroscopic measurement can be performed.

In FIGS. 10A and 10B and FIGS. 11A and 11B, transmitted infrared light is detected by the light-receiving element 35, but it is also applicable to an infrared analysis chip in which reflected infrared light reflected by a sample is detected by the light-receiving element 35. In FIGS. 10A and 10B and FIGS. 11A and 11B, the nanocarbon light sources 25 are arranged in a row along the flow path 23, but it is needless to say that the nanocarbon light sources 25 may be arranged in a two-dimensional manner.

### <Fourth Embodiment>

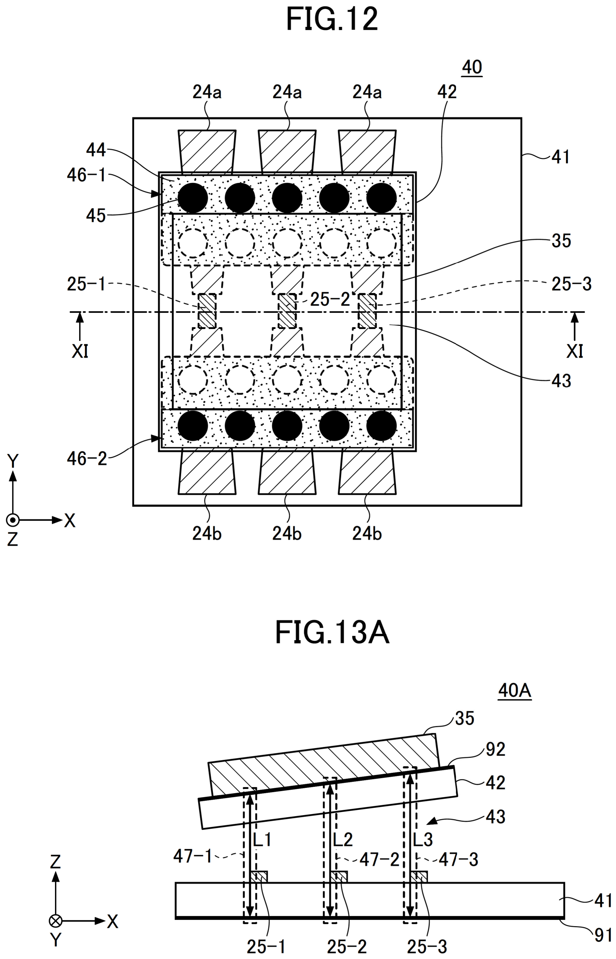

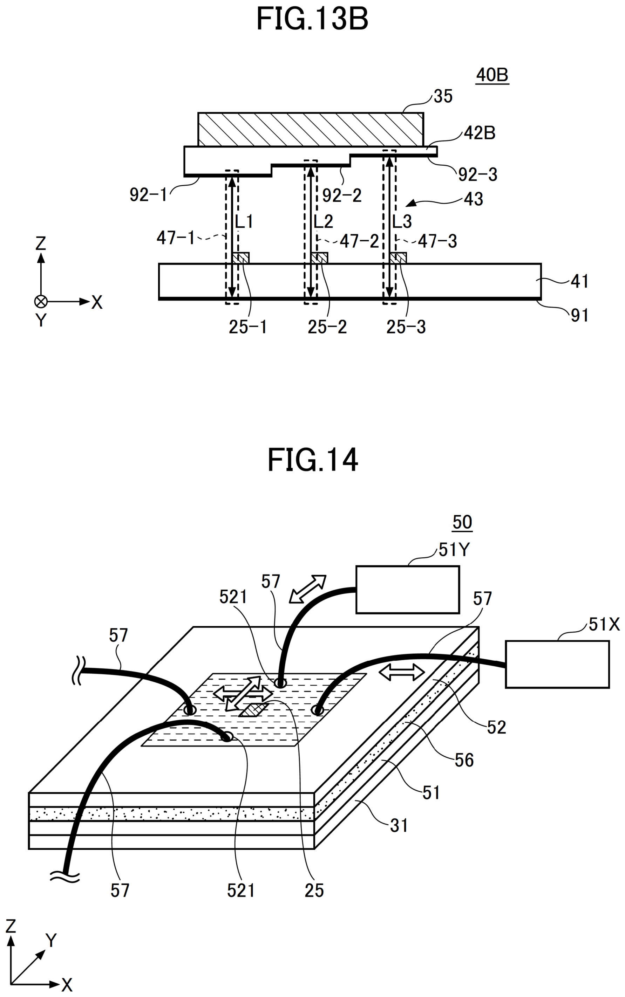

FIG. 12 is a schematic top view of an infrared analysis chip 40 according to the fourth embodiment, and FIGS. 13A and 13B illustrate structural examples of the cross section XI-XI of FIG. 12. In the fourth embodiment, resonators having tunable resonator lengths are provided in the infrared analysis chip 40. The resonator length of each resonator formed between a first substrate 41 and a second substrate 42 is changed by changing the distance between the first substrate 41 and the second substrate 42 according to the position of the nanocarbon light source 25.

In FIG. 13A, the second substrate 42 is tilted relative to the first substrate 41, and the height of the flow path 43 formed between spacers 46-1 and 46-2 is changed along the traveling direction of the flow path. If the height of the light source is changed with respect to the flow path, it can be tilted by any method. In the case where the light sources are also arranged perpendicular to the flow direction of the flow path, the same effect can be obtained in the perpendicular direction by tilting the light sources in that direction as well. For example, the second substrate 42 is tilted with respect to the first substrate 41 in the X direction in which the nanocarbon light sources 25-1, 25-2 and 25-3 are arranged. In order to tilt the second substrate 42 relatively, for example, projections 45 formed on the first substrate 41 or the second substrate 42 are used as a spacer member included in the spacer 46-1 and the spacer 46-2 (sometimes collectively referred to as "spacer 46"). The height of the projections 45 is gradually increased in the arrangement direction (for example, the X direction) of the nanocarbon light sources 25-1, 25-2, and 25-3. An adhesive layer 44 is filled between the projections 45, the second substrate 42 is arranged on the projections and the adhesive layer 44 is cured, thereby obtaining the spacer 46 in which the projections 45 as a spacer member and the adhesive layer 44 are integrated. A flow path 43 whose height changes in the X direction is formed between the spacer 46-1 and the spacer 46-2.

A first mirror 91 having a first mirror surface is provided in a region of the first substrate 41 corresponding to at least the flow path 43. A second mirror 92 having a second mirror surface is provided in a region of the second substrate 42 corresponding to at least the flow path 43. Resonators 47-1, 47-2, and 47-3 respectively having different resonator lengths L1, L2, and L3 are formed between the first mirror 91, and the second mirror 92 corresponding to the nanocarbon light sources 25-1, 25-2, and 25-3, respectively. Since resonators 47-1, 47-2, and 47-3 respectively having different resonator lengths L1, L2, and L3 are provided for the nanocarbon light sources 25-1, 25-2, and 25-3, infrared measurement at a plurality of wavelengths in the infrared region can be performed at one time. The first mirror 91 may be formed on the back surface of the first substrate 41 or on the surface facing the second substrate 42. The second mirror 92 may be formed on the top surface of the second substrate 42 or on the back surface facing the first substrate 41.

In FIG. 13B, at least one of the facing surfaces of the first substrate 41 or the second substrate 42 is formed in a stepped shape. For example, the first mirror 91 is formed on the back surface of the first substrate 41, the facing surface of the second substrate 42 to the first substrate 41 is formed in a stepped shape, and the second mirrors 92-1, 92-2, and 92-3 are arranged on the steps respectively. In FIG. 13B, the distance between the first mirror 91 and the second mirrors 92-1, 92-2, and 92-3 is varied while the first mirror 91 and the second mirrors 92-1, 92-2, and 92-3 are kept parallel to each other. Thus, the resonators 47-1, 47-2, and 47-3 having variable resonator lengths are formed. As described above, the first mirror 91 may be provided on the facing surface of the first substrate 41 to the second substrate 42.

In the infrared analysis chip 40 of fourth embodiment, the nanocarbon light sources 25-1, 25-2, and 25-3 are not required. The configuration in which the resonator length of the infrared analysis chip 40 is tunable can also be applied to the infrared analysis chip of the first embodiment illustrated in FIGS. 1A through 5, which enables efficient multi-wavelength infrared spectroscopic analysis even in the case where an external infrared light source is used.

### <Fifth Embodiment>

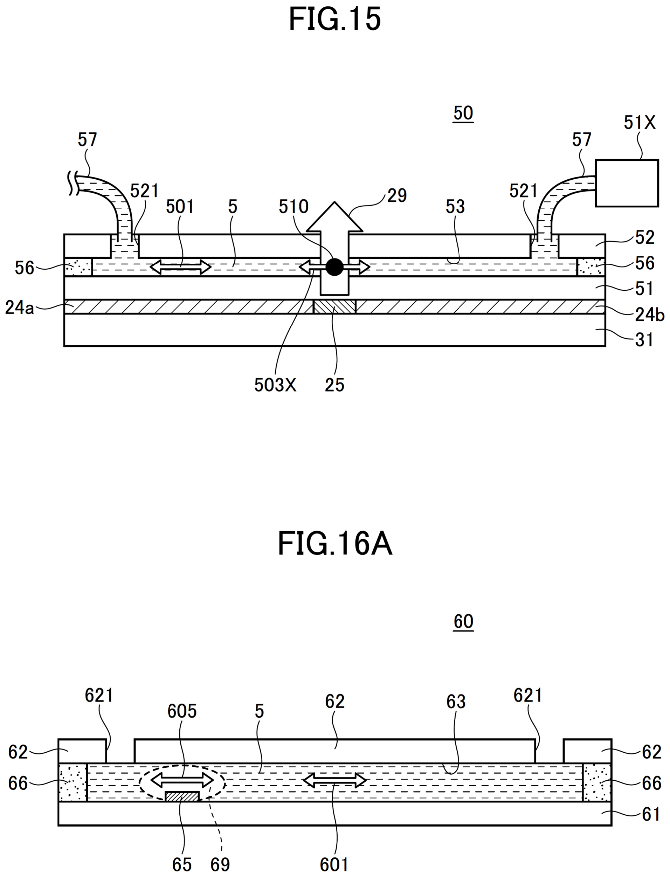

FIG. 14 is a schematic perspective view of an infrared analysis chip 50 of the fifth embodiment, and FIG. 15 is a cross-sectional configuration example of the infrared analysis chip 50 of FIG. 14. The thickness direction of the infrared analysis chip 50 is the Z direction, and the plane perpendicular to the Z direction is the XY plane. The infrared analysis chip 50 enables liquid to be injected into and suctioned from a flow path. The infrared analysis chip 50 includes a first substrate 51, a second substrate 52, a flow path 53 formed between the first substrate 51 and the second substrate 52, and the nanocarbon light source 25 for irradiating the flow path 53 with infrared light. In this example, the flow path 53 is formed between the first substrate 51 and the second substrate 52 by a spacer 56, but any flow path may be used. The nanocarbon light source 25 is formed, for example, on the surface of the third substrate 31 and adhered to the back surface of the first substrate 51 but may be arranged in any configuration as long as it can irradiate the flow path 53. The flow path 53 is connected to a manipulator for injecting or suctioning liquid through a tube 57. Appropriate devices, such as a microinjector, a microaspirator, a micropump, and a syringe, may be used as a manipulator for injecting and suctioning liquid.

In the configuration examples illustrated in FIGS. 14 and 15, a hole 521 connected to the flow path 53 is formed in the second substrate 52, and the tube 57 is inserted into at least a part of the hole 521 and connected to the flow path 53. The periphery of the hole 521 and the tube 57 may be sealed with a resin or the like. The tube 57 is connected, for example, in a direction perpendicular to the center of the flow path 53 and is connected to a microinjector 51X for the X direction and a microinjector 51Y for the Y direction, respectively. By the microinjectors 51X and 51Y, a small amount of liquid 5 can be freely suctioned from or injected into the flow path 53 from the X direction and the Y direction. By causing the liquid 5 to finely flow inside the infrared analysis chip 50 in a specific direction, the position of the liquid in the flow path 53 can be freely controlled. In this way, the microinjectors 51X and 51Y propel the liquid introduced into the flow path 53 in a one-dimensional direction or a two-dimensional direction. The tube 57 may be inserted into the hole 521 in a direction opposite to the hole 521 into which the tube 57 connected to the microinjectors 51X and 51Y is inserted or may be sealed without insertion of the tube 57. Depending on the viscosity of the liquid 5, the hole 521 may be left as it is.

Referring to FIG. 15, by driving the microinjector 51X in the X direction, the liquid passing through the tube 57 moves in the direction of the double-headed arrow 501, and the liquid 5 in the flow path 53 is propelled in the +X or -X direction indicated by the double-headed arrow 501. When the liquid 5 containing a minute substance 510 is subjected to infrared analysis by the nanocarbon light source 25, the minute substance 510 to be analyzed can be freely moved or propelled in the direction of the double-headed arrow 503X to a region irradiated by the nanocarbon light source 25. Unlike a stage usually used in microspectroscopy, a minute measurement target in the liquid 5 can be moved directly above the nanocarbon light source 25 for local analysis or imaging without moving the stage. Since the liquid 5 itself moves, the liquid 5 can be subjected to infrared analysis by the nanocarbon light source 25 or an external light source in a flowing state. Infrared spectroscopy is possible not only by measuring transmitted light 29 but also by detecting reflected light or scattered light from a sample.

The liquid propelling method of the fifth embodiment can be applied to all the infrared analysis chips described in the first through fourth embodiments. The liquid propelling method of the fifth embodiment enables infrared analysis and infrared imaging of a freely chosen minute target, such as local analysis and infrared imaging of all substances including organic and inorganic substances and local analysis and imaging of biological samples, such as cells.

### <Sixth Embodiment>

In the second through fifth embodiments, the thermal insulating layer 28 or a thermally insulating substrate is interposed between the flow path and the nanocarbon light source 25 in order to suppress contact between the nanocarbon light source 25 and the liquid 5. There may be cases, on the other hand, where heat generated by a nanocarbon light source exhibiting blackbody radiation is actively used. In the sixth embodiment, thermal contact between a nanocarbon device and a sample is improved to provide an infrared analysis chip using the nanocarbon device as a nanocarbon heater.

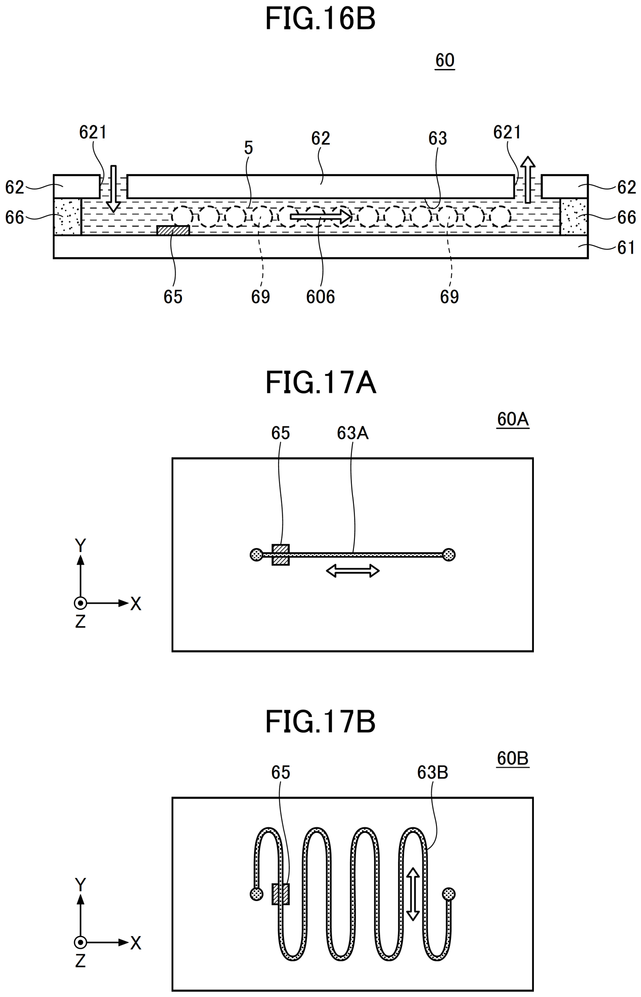

FIGS. 16A and 16B are schematic cross-sectional views of an infrared analysis chip 60 of the sixth embodiment. The infrared analysis chip 60 has a nanocarbon heater 65. FIG. 16A illustrates a configuration example in which the nanocarbon heater 65 is used as a liquid propelling pump, and FIG. 16B illustrates a configuration example in which the nanocarbon heater 65 is used as a bubble generating and transferring heater. The infrared analysis chip 60 has a flow path 63 formed between a first substrate 61 and a second substrate 62, and a nanocarbon heater 65 provided in the flow path 63. In the examples of FIGS. 16A and 16B, the flow path 63 is formed by a spacer 66, but any structure of the flow path may be used. The structure of the nanocarbon heater 65 is the same as that of the nanocarbon light source 25, and the nanocarbon material is energized through a pair of electrodes connected to the nanocarbon material. The nanocarbon heater 65 arranged in the flow path 63 is electrically insulated from liquid 5 introduced into the flow path 63, but the thermal insulation is minimized. The thermal insulation can be minimized by forming a protective film that electrically insulates the nanocarbon heater 65 from the liquid 5 as thin as possible, or by using a protective film that is electrically insulated and has high thermal conductivity without using a thermally insulating protective film. Furthermore, by thermally insulating the nanocarbon heater 65 and the first substrate 61, heat conduction from the nanocarbon heater 65 to the liquid 5 in the flow path 63 is promoted, and the liquid 5 is locally heated.

In FIG. 16A, when the nanocarbon heater 65 is energized, the nanocarbon heater 65 generates heat by black body radiation. The liquid 5 is locally heated by heat from the nanocarbon heater 65, and bubbles 69 are generated in the liquid 5. By increasing or decreasing an applied voltage, the temperature of the nanocarbon heater 65 increases or decreases. As shown by the double-headed arrows 605, by increasing a voltage, the temperature of the liquid 5 increases locally and the bubbles 69 expand, and by decreasing a voltage, the temperature of the liquid 5 decreases locally and the bubbles 69 contract. Using the expansion and contraction of the bubbles 69, the nanocarbon heater 65 and the bubbles 69 can be used as a pump. Since the nanocarbon heater 65 is not used as a light source for infrared analysis, it can be driven at a high temperature. By using the nanocarbon heater 65 as a pump, the liquid in the flow path 63 is propelled in the flow path 63 as shown by the double-headed arrows 601. An opening 621 for introducing liquid provided in the infrared analysis chip 60 may be left as it is after the liquid 5 is injected into the flow path 63, or may be sealed with a resin or the like. A tube or hose for injection may remain inserted into the opening 621.

FIG. 16B illustrates how the liquid 5 is continuously supplied into the flow path 63 from the opening 621. When a constant voltage is applied to the nanocarbon heater 65, it generates heat at a predetermined temperature by blackbody radiation. When the supplied liquid 5 thermally contacts the nanocarbon heater 65, bubbles 69 are sequentially generated. The generated bubbles move on the liquid 5 flowing in the flow path 63. By using the nanocarbon heater 65 as a microheater for bubble generation/transfer, the bubbles 69 are transferred in the direction of the arrow 606. By generating the bubbles 69 while flowing the liquid in the flow path 63, a flow of many bubbles can be created along the flow path 63. As a feature of this configuration, since only local heating is performed by the nanocarbon heater 65, the temperature of the liquid 5 in the flow path 63 barely increases, and the bubbles 69 can be sent into the unheated liquid 5. In conventional macroscopic heaters, the entire liquid is heated, and bubbles cannot be sent one after another into unheated liquid. With this mechanism of the present embodiment, the device can generate microbubbles one after another in the flow path.

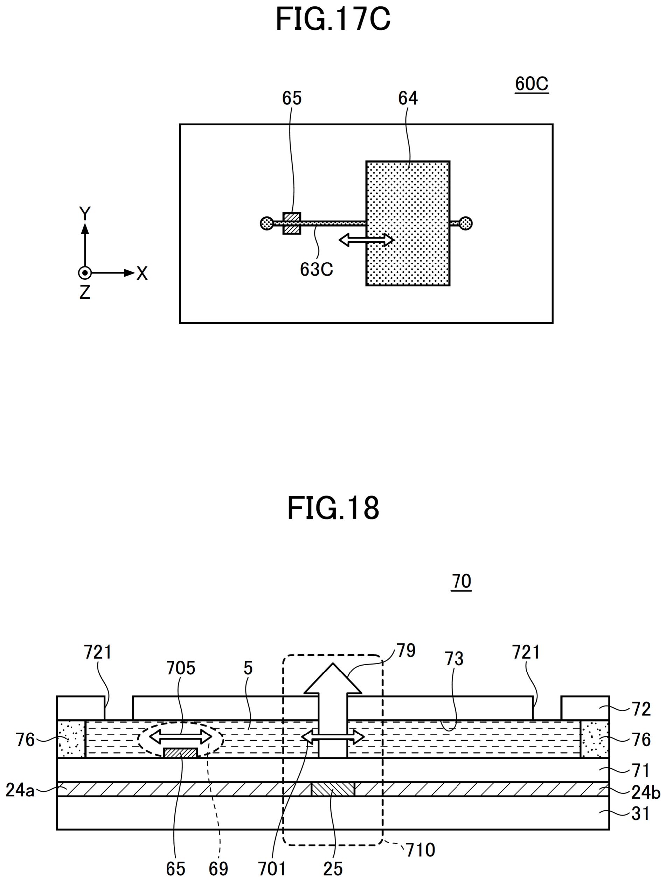

FIGS. 17A, 17B, and 17C are diagrams illustrating a modified example of the infrared analysis chip 60 according to the sixth embodiment. In FIG. 17A, an infrared analysis chip 60A has a flow path 63A extending in one direction. The nanocarbon heater 65 as a heat source is arranged near one end of the flow path 63A. When the nanocarbon heater 65 is energized to raise or lower the voltage level, it functions as a pump for propelling liquid as illustrated in FIG. 16A. As illustrated in FIG. 16B, the nanocarbon heater 65 may function as a microheater for generating and transferring bubbles by continuously supplying liquid 5 to the flow path 63A to generate heat at a constant temperature. In FIGS. 17A, 17B, and 17C, the nanocarbon heater 65 is depicted larger than the width of the flow paths 63A through 63C for visibility; in reality, the nanocarbon heater 65 may be smaller or larger than the flow paths 63A through 63C.

In FIG. 17B, an infrared analysis chip 60B has a meander-shaped flow path 63B. The nanocarbon heater 65 as a heat source is arranged at a suitable location of the flow path 63B. By energizing the nanocarbon heater 65 and controlling the voltage level or an on/off state, the nanocarbon heater functions as a pump for propelling the liquid or a microheater for generating and transferring bubbles. In FIG. 17C, the infrared analysis chip 60C has a flow path 63C and a reservoir 64 connected to the flow path 63C. In the case where the flow path is formed by the spacer structure of the first embodiment, the reservoir 64 having a wide space can be easily formed simultaneously with the flow path 63C. By storing liquid 5 in the reservoir 64 and driving the nanocarbon heater 65, the liquid 5 can be propelled along the flow path 63C. The configurations of FIGS. 17A, 17B, and 17C may be combined with each other. Similarly to the nanocarbon light source 25, the nanocarbon heater 65 may be arranged in a one- or two-dimensional array and sequentially driven to control the place where bubbles are generated.

In the sixth embodiment, by using the nanocarbon heater 65, liquid can be propelled without using an external liquid injection/suction device such as a microinjector. The nanocarbon heater 65 is heated to a minimum of about 10 °C and a maximum of about 2,000 °C to serve as a local high temperature microheater. Bubbles 69 are formed by locally vaporizing a liquid sample, and the liquid 5 can be propelled by using the expansion and contraction of the bubbles 69. In the case where the liquid 5 is propelled or transferred by the bubbles 69 generated by the nanocarbon heater 65, an external microinjector, a pump, a syringe, or the like are unnecessary, and the infrared analysis chip 60 in which the flow of a fluid is controlled with a simple configuration is realized. Like the nanocarbon light source array of the second embodiment, a plurality of nanocarbon heaters 65 may be arranged in an array shape to perform local heating at a plurality of places.

It is possible to apply the configuration of the sixth embodiment not only to an infrared analysis chip but also to a general-purpose microflow path device. It is possible to use the configuration of the sixth embodiment as a micropump for controlling fluid only by forming the nanocarbon heater 65 in the flow path 63. The planar arrangement of the flow path 63 is not particularly limited. The infrared analysis chip 60 is not limited to a flat rectangular chip, and a tube-type flow path device extending long in a one-dimensional direction or a flow path device having a three-dimensional structure to which the nanocarbon heater 65 is added as a pump may be used.

Unlike other heaters using metal filaments or wires, the nanocarbon heater 65 has a very small shape and a small heat capacity, and functions that cannot be realized with other material systems are obtained. For example, since it has a minimum size of nanometer order, it is possible to heat liquid in a local area of a minimum nanometer order, typically 1 µm or more and less than 1 mm, for example, 10 µm or more and 500 µm or less. Since only a part of the flow path can be locally heated, only a part of the liquid can be vaporized, which is a feature unique to the embodiment that is difficult to realize with ordinary macroscopic metal filaments. Since a large area of the flow path is heated when using a macroscopic heater, formation of bubbles by local heating is also not possible, and it cannot be used as a micropump.

Since the nanocarbon heater 65 can be formed in a freely chosen shape at a freely chosen position using lithography technology on various substrates, it can be used as a microheater or a micropump by local heating, which is difficult to realize with other materials. Since nanocarbon materials are chemically stable compared with conventional metal heaters, it is a major feature that the heater does not appreciably deteriorate due to chemical reactions with a sample liquid or external environment. This is due to the fact that nanocarbon materials have a two-dimensional graphene crystal structure as a basic structure and do not have dangling bonds on their surface, making them chemically stable. Nanocarbon materials are highly durable compared with other metal and semiconductor materials.

The nanocarbon heater 65 is a very thin layer with a thickness of 0.3 nm at the minimum and 100 µm at the maximum, so it has a very small heat capacity and enables heating and cooling at a fast rate. Typically, a thickness of about 0.3 nm to 10 µm is used, and about 10 nm to 1 µm is used as a suitable condition, but in any case, it has a small heat capacity and enables heating and cooling at a fast rate. For example, it is possible to control the heating and vaporization of liquid by fast heating, and to control the expansion and contraction of bubbles at a fast rate, and it is possible to control the flow of fluids with high precision, which cannot be realized by conventional heaters with a large heat capacity. The nanocarbon heater 65 can be heated using direct current, or it may also be used with modulated power. In the latter case, it is possible to modulate a current at 1 Hz or greater, and it is possible to modulate a current at a high modulation rate of 10 Hz or greater, which is difficult with conventional heaters, and it is possible to heat at a rate of 100 Hz, 1 kHz, or 10 kHz, which is normally possible, and an ultra-thin nanocarbon heater of about 1 atomic layer can heat at a rate of about 1 GHz. Since it is possible to heat and cool at a fast rate, it is possible to control heating by rectangular waves or pulses. For example, precise heating control and temperature control by changing the duty ratio in a rectangular wave, and ultrafast heating and cooling control by pulse control become possible, and high performance, precise and various temperature control become possible. As for the pulse width, pulse control with a time width of about the inverse of the above-mentioned modulation rate is possible, and for example, operation with a pulse width of about 1 seconds is possible, but narrow pulse widths from 100 milliseconds to 1 nanosecond are also possible, and typically pulse widths of the order of 10 milliseconds, 1 millisecond, and 100 microseconds (µs) become possible.

### <Seventh Embodiment>

In the seventh embodiment, the nanocarbon light source 25 and the nanocarbon heater 65 are mounted on one chip to realize more flexible infrared analysis and measurement. The nanocarbon light source 25 and the nanocarbon heater 65 may have the same structure as a nanocarbon device.

FIG. 18 is a schematic cross-sectional view of an infrared analysis chip 70 according to the seventh embodiment. The infrared analysis chip 70 includes a flow path 73 into which liquid 5 to be measured is introduced, the nanocarbon light source 25 for irradiating the flow path 73 with infrared light, and the nanocarbon heater 65 for locally heating the liquid 5 introduced into the flow path 73. The nanocarbon light source 25 is thermally and electrically insulated from the liquid 5 introduced into the flow path 73. On the other hand, thermal insulation between the nanocarbon heater 65 and the liquid 5 is minimized.

In the configuration example of FIG. 18, the flow path 73 is formed between a first substrate 71 and a second substrate 72 by a spacer 76, and an opening 721 for introducing or discharging liquid is formed in the second substrate 72, but the configuration is not limited to this flow path configuration. Any flow path configuration may be used as long as a sample introduced by the nanocarbon light source 25 is irradiated and the nanocarbon heater 65 can be provided inside the flow path. The nanocarbon light source 25 is provided, for example, on the back surface of the first substrate 71 and is separated from the flow path 73 to ensure thermal and electrical insulation from the liquid 5. The nanocarbon heater 65 is provided inside the flow path 73 and is electrically insulated from the liquid 5, but the thermal insulation from the liquid 5 is minimized. The entire infrared analysis chip 70 including the flow path 73, the nanocarbon light source 25, and the nanocarbon heater 65 may be supported on the third substrate 31.

Bubbles 69 are generated by energizing the nanocarbon heater 65 and locally heating the liquid 5. By repeating heating and cooling locally by raising and lowering the voltage level applied to the nanocarbon heater 65, the bubbles 69 are repeatedly expanded and contracted as illustrated by the double-headed arrow 705. By this pumping operation, the liquid 5 is propelled in the flow path 73 as shown by the double-headed arrow 701. By the liquid 5 moving in the analysis region 710 provided with the nanocarbon light source 25, the liquid 5 or a minute substance contained in the liquid 5 is irradiated by an analysis light 79 and measured. By combining the infrared analysis with the local heating technique, the liquid 5 itself introduced into the flow path 73 and the minute substance contained in the liquid 5 are propelled to the position of the nanocarbon light source 25, and the infrared analysis can be measured efficiently.

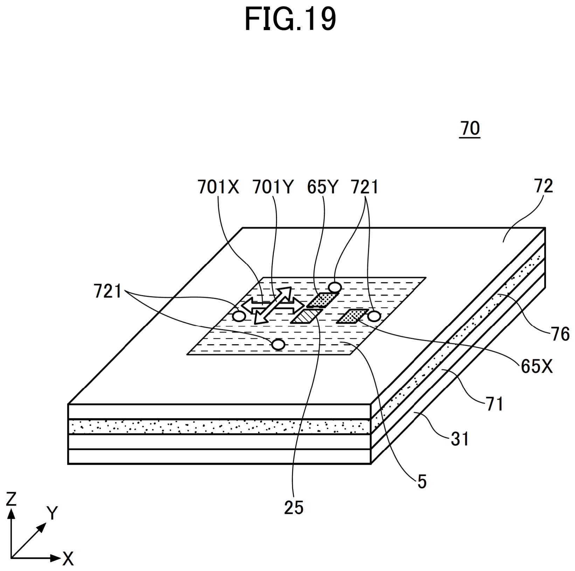

FIG. 19 illustrates an example of the arrangement of the nanocarbon light source 25 and the nanocarbon heater 65 of the infrared analysis chip 70. The infrared analysis chip 70 is provided with a nanocarbon heater 65X for propelling the liquid 5 in the X direction and a nanocarbon heater 65Y for propelling the liquid 5 in the Y direction. By driving the nanocarbon heater 65X or 65Y, the liquid 5 is propelled in the direction of the double-headed arrow 701X or 701Y, and the propelling of the liquid 5 can be controlled two-dimensionally. Since the liquid 5 can be freely propelled in two dimensions, heating by mapping onto a plane is also possible. It is thereby possible to achieve local heating of all materials, such as organic and inorganic materials, and local heating of biological samples, such as cells.

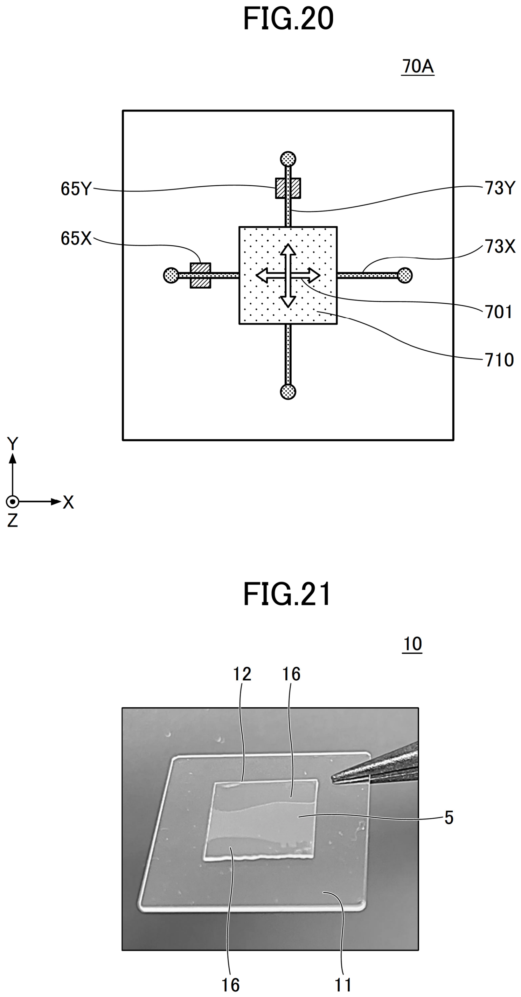

FIG. 20 is a schematic diagram of an infrared analysis chip 70A to illustrate another arrangement example. The infrared analysis chip 70A has a flow path 73X extending in the X direction and a flow path 73Y extending in the Y direction, and an analysis region 710 for analysis using infrared light irradiation is provided in a region where the flow paths 73X and 73Y intersect. The nanocarbon heater 65X is provided in the flow path 73X to propel the liquid in the flow path 73X in the X direction. The nanocarbon heater 65Y is provided in the flow path 73Y to propel the liquid in the flow path 73Y in the Y direction. With this configuration, the liquid 5 is also propelled in the direction of the double-headed arrow 701X or 701Y, and the propelling of the liquid 5 can be controlled two-dimensionally. Since the liquid 5 can be freely propelled in two dimensions, heating by mapping is also possible. It is thereby possible to achieve local heating of all materials, such as organic and inorganic materials, and local heating of biological samples, such as cells.

### <Manufacture of Infrared Analysis Chip and Measurement Results>

FIG. 21 is an image of the infrared analysis chip 10 of the first embodiment actually manufactured. The second substrate 12 is arranged on the first substrate 11, and liquid 5 is introduced between the first substrate 11 and the second substrate 12 and between the spacers 16. The spacers 16 are obtained by integrating particles and an adhesive layer by curing. The size of the first substrate 11 is 20 mm × 20 mm × 0.5 mm, the size of the second substrate 12 is 10 mm × 10 mm × 0.5 mm, and the width between the spacers 16 is 3 to 6 mm. Infrared measurement of a sample can be easily performed by irradiating the infrared analysis chip 10 with infrared light from an external infrared light source and measuring transmitted light through liquid 5.

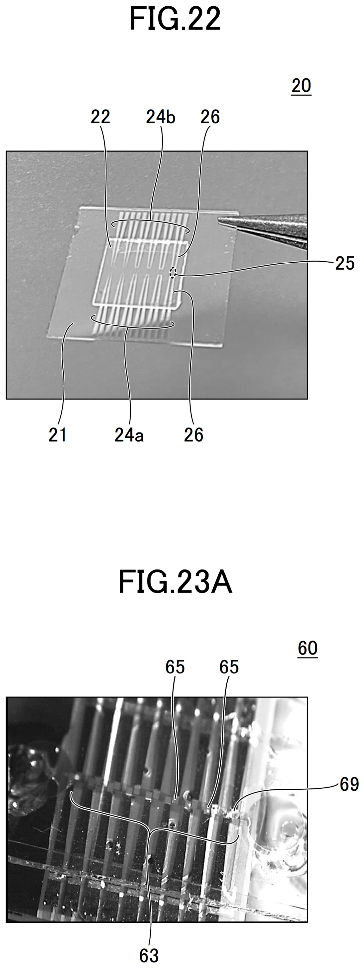

FIG. 22 is an image of the infrared analysis chip 20 provided with the nanocarbon light sources 25 arranged in an array. The electrodes 24a and 24b connected to the nanocarbon light source 25 are also formed on the first substrate 21. Liquid is introduced between the first substrate 21 and the second substrate 22 and between the spacers 26. The size of the first substrate 21 is 20 mm × 20 mm × 0.5 mm, the size of the second substrate 22 is 10 mm × 10 mm × 0.5 mm, the space between the pair of spacers 26 is 2 to 3 mm, and the size of the nanocarbon light sources 25 formed on the first substrate 21 is one of the following three types: 5 µm × 5 µm, 10 µm × 10 µm, and 20 µm × 20 µm. As illustrated in FIGS. 21 and 22, compact-sized infrared analysis chips 10 and 20 capable of easily performing infrared measurement with a simple configuration are realized.

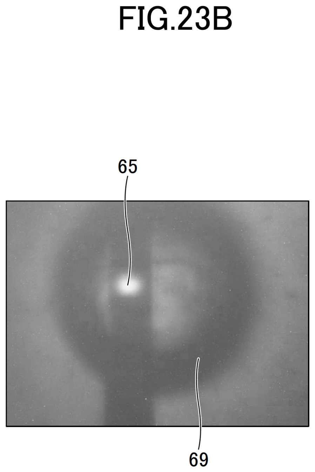

FIGS. 23A and 23B are images of bubble generation in a flow path in the infrared analysis chip 60 of the sixth embodiment. In FIG. 23A, the nanocarbon heaters 65 are arranged in an array in the flow path 63. In this figure, each nanocarbon heater 65 is formed of graphene. It can be observed that water evaporates and the bubbles 69 expand to push out the fluid when the rightmost nanocarbon heater 65 is energized and heated. FIG. 23B is an image illustrating the result of generating minute bubbles 69 one after another on the nanocarbon heater 65 by using the nanocarbon heater 65 of FIG. 23A, and causing the bubbles 69 to flow along the flow path. It can be understood that by controlling the generation or expansion and contraction of the minute bubbles 69 by using the infrared analysis chip 60 of the sixth embodiment, propelling or transferring the sample liquid can be realized without an external device.

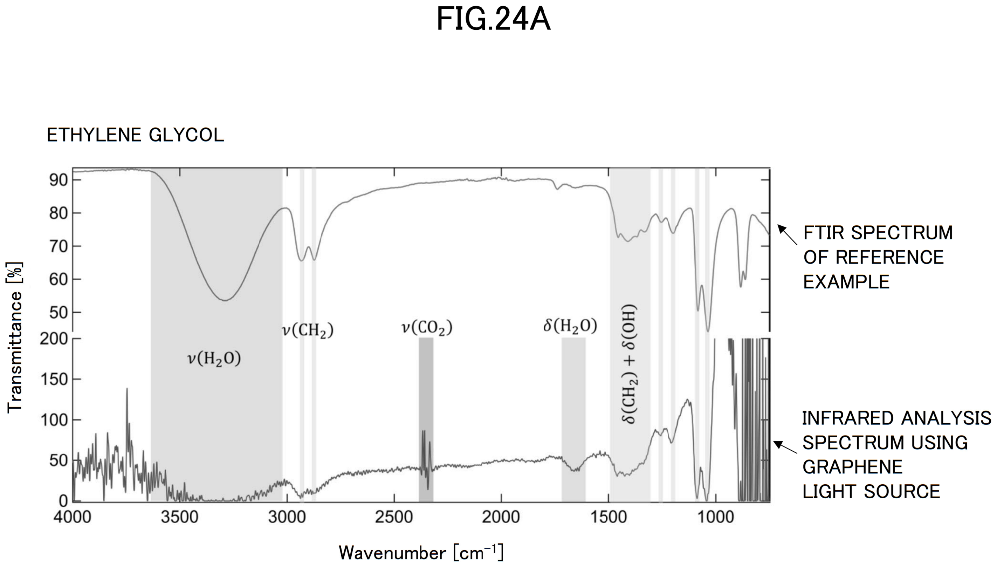

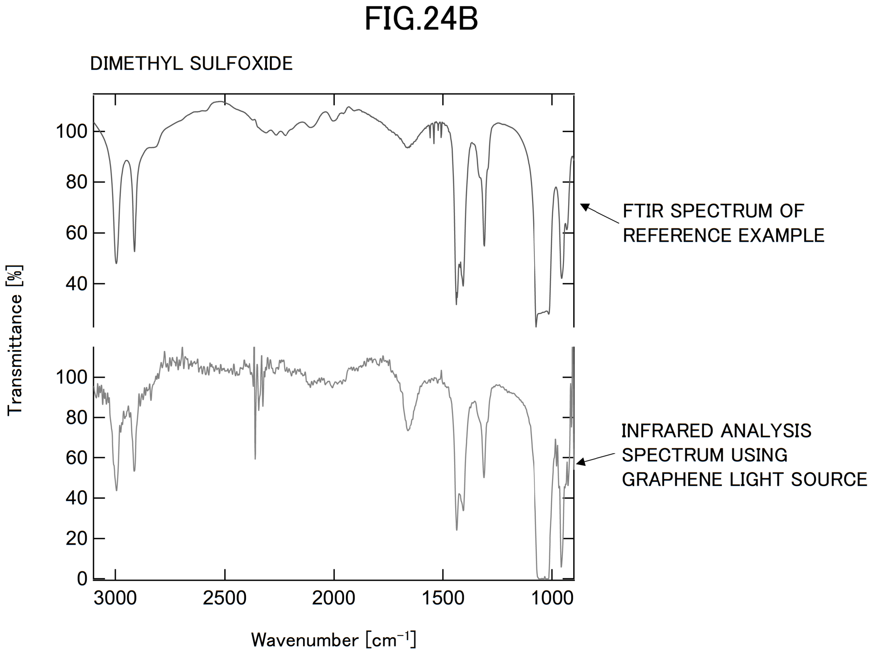

FIG. 24A is a graph showing an infrared absorption spectrum of ethylene glycol measured with the infrared analysis chip 30 of FIGS. 10A and 10B. FIG. 24B is a graph showing an infrared absorption spectrum of dimethyl sulfoxide measured with the infrared analysis chip 30 of FIGS. 10A and 10B. Specifically, these are an absorption spectrum of ethylene glycol and an absorption spectrum of dimethyl sulfoxide, both obtained by introducing a liquid sample into the flow path 23 formed above the nanocarbon light sources 25 in an array and performing infrared measurement of the liquid sample with the nanocarbon light sources 25 and the light-receiving element 35. The horizontal axis is a wave number (cm⁻¹), and the vertical axis is a transmittance, and an FTIR spectrum measured by a general method is also shown. With the simple and compact infrared analysis chip 30 illustrated in FIGS. 10A and 10B, it is possible to reliably analyze and measure a trace amount of liquid chemical substances, such as ethylene glycol and dimethyl sulfoxide, with the same accuracy as the infrared analysis by FTIR using the macro light source of a reference example.

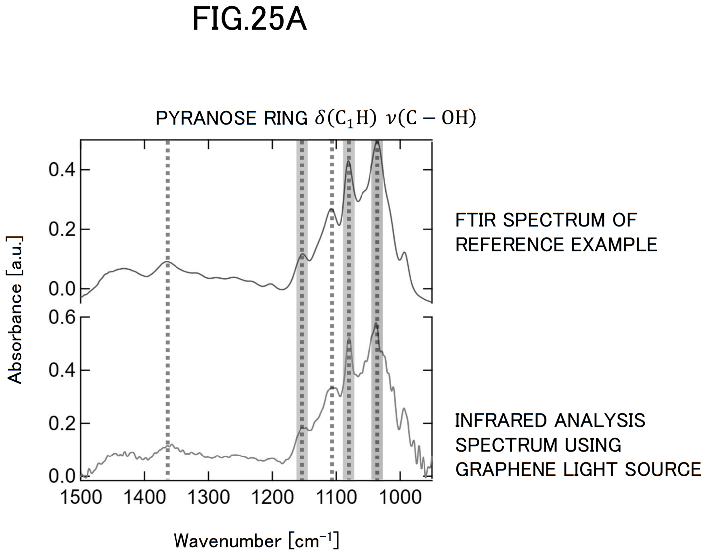

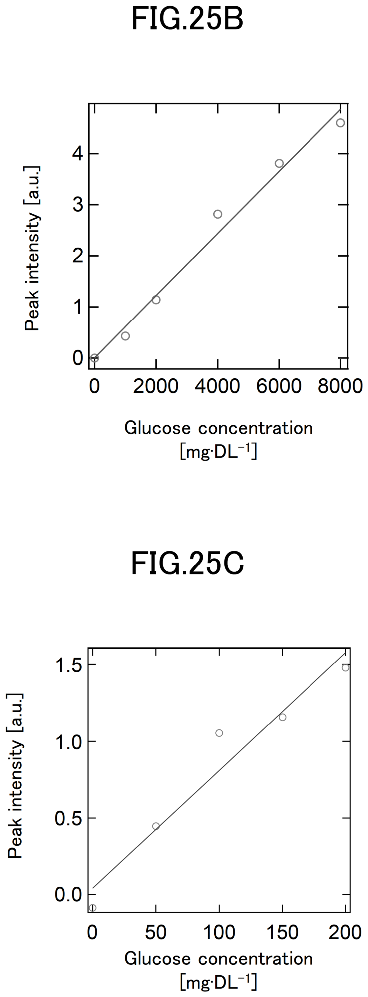

FIGS. 25A through 25C illustrate the results of measuring glucose using the infrared analysis chip 30 illustrated in FIGS. 10A and 10B. FIG. 25A is an infrared absorption spectrum of glucose, FIG. 25B is a diagram illustrating the correlation between glucose concentration and peak intensity, and FIG. 25C is a diagram illustrating the correlation between glucose concentration and peak intensity in a low concentration region. As illustrated in FIG. 25A, it is verified that the infrared analysis chip 30 can measure glucose with the same accuracy as conventional macro FTIR infrared analysis. As illustrated in FIGS. 25B and 25C, calibration curves for glucose concentration are also obtained, and it is understood that the concentration of glucose can be measured using the infrared analysis chip 30. Since the quantitative analysis of glucose can be applied to a glucose monitor in the diagnosis of blood glucose levels due to diabetes, it was verified that the nanocarbon light source 25 can be applied to blood glucose monitoring for diabetes.

### <Eighth Embodiment>

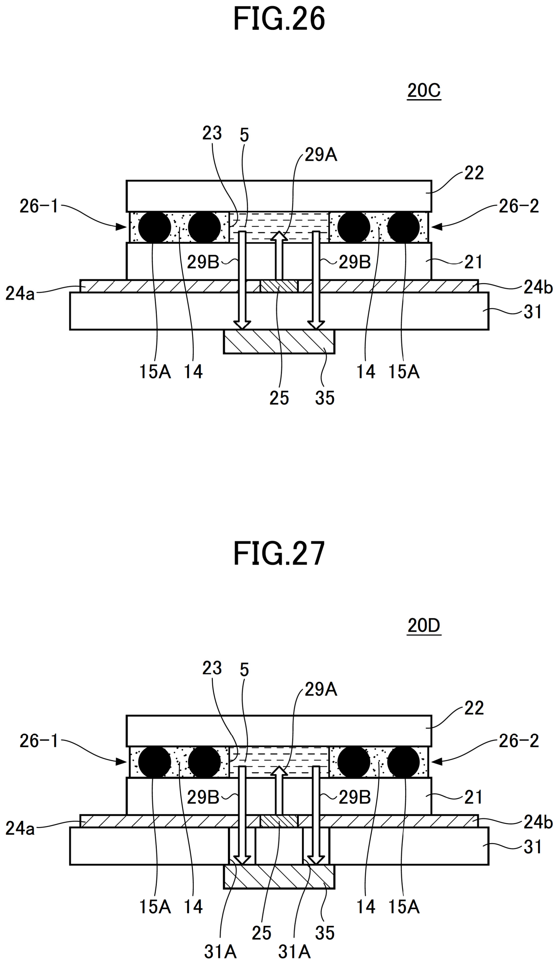

The infrared analysis chips of the first through seventh embodiments are capable of measurement using not only transmitted infrared light but also reflected infrared light and scattered infrared light from a sample. FIG. 26 is a schematic cross-sectional view of an infrared analysis chip according to the eighth embodiment. In an infrared analysis chip 20C, the light-receiving element 35 is provided on the back side of the third substrate 31. For example, the first substrate 21 is formed of a material having a high refractive index and transparent to infrared light. An evanescent field is generated at the interface between liquid 5 and the first substrate 21 by irradiated infrared light 29A irradiated to the liquid 5 from the nanocarbon light source 25. Due to the evanescent field, the substance to be analyzed contained in the liquid 5 strongly absorbs infrared light. The light acting on the substance to be analyzed is reflected or scattered by the substance and sent toward the third substrate 31, and reflected infrared light or scattered infrared light 29B is generated. The light-receiving element 35 detects the reflected infrared light or scattered infrared light 29B. The high-refractive-index material used for the first substrate 21 is a material having a refractive index higher than that of the liquid 5 introduced into the flow path 23. The refractive index of the first substrate 21 is a material having a specific refractive index with respect to the wavelength of the infrared light used for analysis larger than 1.4, preferably larger than 1.7, more preferably larger than 2.0, or may be about 4.0. Although Ge and diamond may be used as the high-refractive-index material, any material may be used as long as it transmits infrared light having a wavelength used for analysis. Other configurations are the same as those in FIG. 9.

FIG. 27 is a schematic cross-sectional view of an infrared analysis chip according to a modified example of the eighth embodiment. In an infrared analysis chip 20D, the third substrate 31 is formed of a material that absorbs infrared light having a wavelength used for analysis. The third substrate 31 has a hole or space 31A. The hole or space 31A is provided so as to overlap the flow path 23 and not overlap the nanocarbon light source. The hole or space 31A may be provided near the nanocarbon light source 25. The hole or space 31A may be a through hole penetrating the third substrate 31, a recess formed on the upper or lower surface of the third substrate 31, or a cavity formed in the third substrate 31. The part where the hole or space 31A is formed in the third substrate 31 may have a higher transmittance of infrared light of a wavelength used for analysis than the part where the hole or space 31A is not formed. The absorptance of infrared light of a wavelength used for analysis in the third substrate 31 is preferably 50% or more. More preferably, it is 80% or more, and more preferably, it is 90% or more.

In the infrared analysis chip 20D, since infrared light emitted from the back surface of the nanocarbon light source 25 is absorbed by the third substrate 31, the infrared light does not appreciably reach the back surface side of the third substrate 31. For this reason, even if an optical filter or a blocking layer is not used, light emitted from the back surface of the nanocarbon light source 25 can be cut by the third substrate 31 itself acting as a filter. The reflected infrared light or scattered infrared light 29B passes through the hole or space 31A and reaches the light-receiving element 35. Therefore, the light-receiving element 35 can detect only the reflected infrared light or scattered infrared light 29B from the liquid 5. As the third substrate 31 that absorbs infrared light used for analysis, an inorganic substrate made of, for example, quartz, silicon oxide, glass, sapphire, semiconductor, insulator, or metal, or an organic substrate made of, for example, polymer resin, can be used. The third substrate 31 may be made of a material such as CaF₂, BaF₂, or Ge, Si, KBr, KSR-5, NaCl, ZnSe, ZnS, CsI, SiO₂, or Al₂O₃ that transmits a measurement target wavelength, and it is provided with a blocking layer to absorb infrared light of a wavelength used for analysis.

A mirror or the like reflecting infrared light used for analysis may be provided inside or outside the second substrate 22. Thus, light that passes through the flow path 23 is reflected by the mirror or the like and sent to the back surface of the third substrate 31 through the first substrate 21 and the third substrate 31 (or the hole or space 31A of the third substrate 31). The light-receiving element 35 can also analyze liquid by detecting this infrared light sent to the back surface of the third substrate 31.

### <Ninth Embodiment>

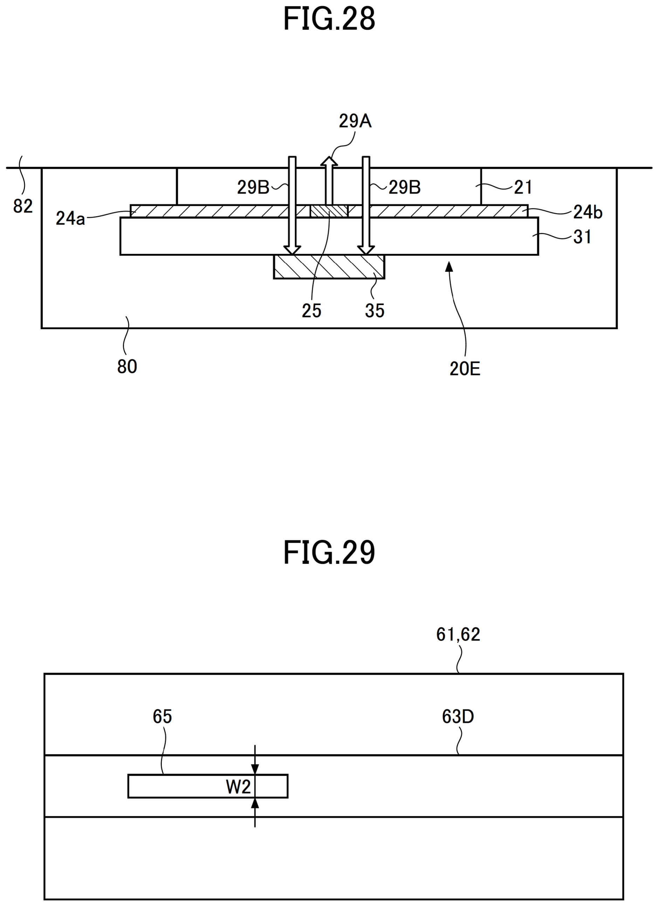

The ninth embodiment is an example of a wearable device. FIG. 28 is a schematic cross-sectional view of a wearable device according to the ninth embodiment. A wearable device 80 includes an infrared analysis chip 20E. The infrared analysis chip 20E does not include the second substrate 22, the flow path 23, and the spacers 26-1 and 26-2 in contrast to the infrared analysis chip 20C of the eighth embodiment. When the wearable device 80 is used, the surface of the first substrate 21 opposite to the third substrate 31 is brought into direct contact with a living body 82 such as skin of a human body. The wearable device 80 uses the infrared analysis chip 20E to measure substances contained in blood or sweat. The hole or space 31A may be provided in the third substrate 31 as in the infrared analysis chip 20D in the modified example of the eighth embodiment.

In the ninth embodiment, an evanescent field generated at the interface between the first substrate 21 having a high refractive index and the living body 82 makes it possible to perform contact-type infrared analysis or sensing to the living body 82. Up to now, LEDs (light emitting diodes), which are semiconductor light sources, have been mainly used for such contact-type infrared analysis, and it has not been possible to use infrared light sources having a wide emission wavelength in mid-infrared. To realize the principle of contact-type infrared analysis and measurement using a conventional mid-infrared light source, it is necessary to use a thermal light source, such as a halogen lamp. However, the halogen lamp is vacuum-sealed by a glass tube or the like. It is therefore impossible to directly bond the light source lamp with a high-refractive-index transparent substrate or to integrate the light source lamp on a substrate. The nanocarbon light source 25 on the other hand can be directly formed on various substrates and can be integrated on a planar substrate. The ninth embodiment can be applied to any device, such as a smartwatch, a wearable device, a smartphone, or a handy device.

As in the ninth embodiment, the infrared analysis chip 20E is brought into contact with, for example, a living body such as skin of a human body to measure blood vessels, sweat, and other secretions of the human body, thereby realizing a compact infrared sensing or analysis device. For example, since glucose can be measured, the wearable device 80 can be used as a wearable health care device, a blood sugar measuring device, or a continuous monitoring device for diabetes. Since measurement can be performed on skin, the condition of skin can be monitored by measuring substances making up the skin. By measuring substances contained in sweat, it is possible to perform infrared analysis of biomarkers that indicate physical condition or disease. Thus, a health care monitor for monitoring physical condition and disease can be realized. By measuring interstitial fluid present directly under the skin, markers indicating physical condition and disease can be subjected to infrared analysis. Thus, a health care monitor for monitoring physical condition and disease can be realized. By analyzing blood immediately above a blood vessel or capillary, markers indicating physical condition or disease in blood can be subjected to infrared analysis. Thus, a health care monitor for monitoring physical condition and disease can be realized.

In the first through ninth embodiments, the nanocarbon light source 25 made of carbon nanotubes or graphene is a device that emits infrared light by being heated by applying an electric current. Generally, in the nanocarbon light source 25, a patch-shaped nanocarbon such as a square is arranged on a substrate, and an electrode connected to the nanocarbon is formed. In the case of a patch-shaped light emitting surface, because the light-emitting surface is flat and wide, the electric current tends to disperse and bypass certain areas, which can make it difficult to achieve high brightness or uniform illumination. Therefore, the nanocarbon light source may be formed in a thin line shape to intensively apply an electric current.

In the nanocarbon light source having a thin line shape, dispersion or diversion of the electric current is suppressed, and high brightness and uniform light emission can be achieved. Since the quality of the nanocarbon can be improved by forming the light source in a thin line shape, uniformity and yield of the light source can be increased. The thin line configuration may be a meander shape or a stripe shape. Both the meander shape and the stripe shape nanocarbon light sources can be arranged in an array on a substrate and can be multicolored in combination with filters and resonators. In all embodiments, any configuration may be used as a light-receiving element for the infrared light transmitted, reflected, or scattered by the sample as long as the infrared light can be received, and conventional semiconductor detectors, bolometers, pyroelectric elements, thermopiles, and the like can be used. As illustrated in FIG. 6A, a line width W1 of the nanocarbon light source 25 is preferably equal to or greater than 1 µm and equal to or less than 100 µm, more preferably equal to or greater than 2 µm and equal to or less than 50 µm.

It is also preferable that the nanocarbon heater 65 in the sixth and seventh embodiments be thin. The thin line configuration may be a meander shape or a stripe shape. FIG. 29 is a top view of the nanocarbon heater 65 and its vicinity in a modified example of the sixth embodiment and a modified example of the seventh embodiment. As illustrated in FIG. 29, a line width W2 of the nanocarbon heater 65 is smaller than a line width of a flow path 63D. The line width W2 of the nanocarbon heater 65 is preferably equal to or greater than 1 µm and equal to or less than 100 µm, more preferably equal to or greater than 2 µm and equal to or less than 50 µm. This enables high output and uniform heat generation.

### <Tenth Embodiment>

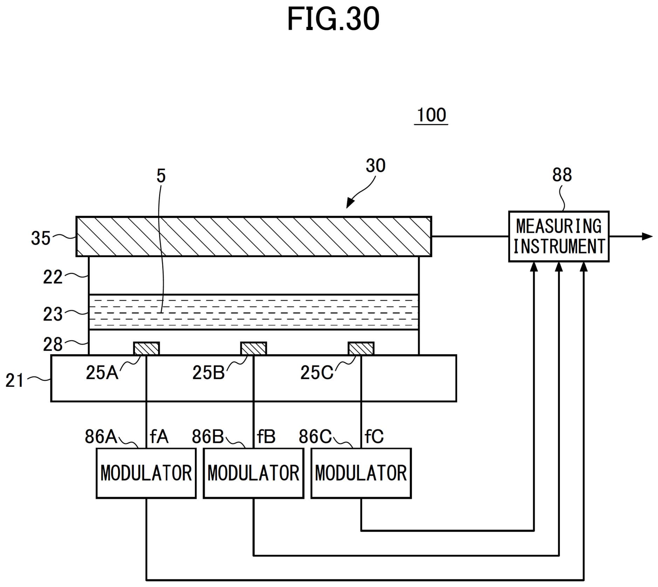

The tenth embodiment is an example of an infrared analysis apparatus. FIG. 30 is a schematic view of an infrared analysis apparatus according to the tenth embodiment. As illustrated in FIG. 30, the infrared analysis apparatus 100 includes the infrared analysis chip 30, modulators 86A through 86C, and a measuring instrument 88. The infrared analysis chip 30 is, for example, an infrared analysis chip according to the third embodiment. The infrared analysis chip may be an infrared analysis chip according to an embodiment other than the third embodiment.

A plurality of nanocarbon light sources 25A through 25C irradiate the flow path 23 with infrared light. The modulators 86A through 86C modulate voltages or currents driving the plurality of nanocarbon light sources 25A through 25C at different frequencies fA to fC, respectively. For example, intensities of infrared light irradiated by the nanocarbon light sources 25A through 25C are modulated at frequencies fA to fC, respectively. The light-receiving element 35 receives infrared light transmitted through the flow path 23. The measuring instrument 88 measures the output signal of the light-receiving element 35 in synchronization with at least one of frequencies fA to fC.

For example, the plurality of nanocarbon light sources 25A through 25C irradiate the flow path 23 with infrared light having different properties. The different properties are, for example, the wavelength of the infrared light and the intensity of the infrared light. When the measuring instrument 88 selects a frequency to be synchronized with the output signal of the light-receiving element 35 from the frequencies fA to fC, liquid 5 can be analyzed using the irradiation light from the nanocarbon light source corresponding to the selected frequency.

The measuring instrument 88 synchronizes the plurality of frequencies fA to fC with the output signal of the light-receiving element 35. This makes it possible to simultaneously analyze the signals synchronized with the frequencies fA to fC using infrared light of distinctive characteristics. The infrared light emitted from the plurality of nanocarbon light sources 25A to 25C may have the same characteristics. Thus, for example, it is possible to simultaneously analyze the liquid 5 at distinctive positions in the flow path 23.

As in the eighth embodiment, the light-receiving element 35 may be provided on the back side of the third substrate 31 and receive the infrared light reflected or scattered from the liquid 5 in the flow path 23.

As described above, according to the present invention, a compact infrared analysis chip capable of easily performing various infrared analyses and measurements can be manufactured at a low cost. The infrared analysis chip of the embodiment is disposable or reusable, and the measurement cost can be kept low. The infrared analysis chip incorporating a nanocarbon light source can easily realize the measurement of a small amount of liquid, the infrared analysis of a small part, and the ultra-compact spectroscopic analysis chip without a need of using a spectrometer. The infrared analysis chip incorporating a light-receiving element together with an array of nanocarbon light sources serves as a compact spectroscopic device capable of performing spectroscopy with a single detector without using a spectrometer using a Michelson interference system or a diffraction grating required for ordinary infrared analysis. The infrared analysis chip of the embodiment enables not only transmission type spectral analysis but also reflection or scattering type spectral analysis, and can be applied to new technologies, such as spectroscopic imaging devices.

The above disclosure includes the following aspects.
(Clause 1) An infrared analysis chip, including:
   a first substrate;
   a second substrate facing the first substrate;
   a flow path provided between the first substrate and the second substrate; and
   one or more nanocarbon light sources thermally insulated from the flow path and configured to irradiate the flow path with infrared light.
(Clause 2) The infrared analysis chip according to Clause 1, wherein
   the one or more nanocarbon light sources are a plurality of nanocarbon light sources, and
   the plurality of nanocarbon light sources are different in size, emission intensity of the infrared light, wavelength of the infrared light, modulation rate of the infrared light, modulation frequency of the infrared light, or emission timing.
(Clause 3) The infrared analysis chip according to Clause 1 or 2, wherein
   the one or more nanocarbon light sources are provided on a surface of the first substrate that faces the second substrate, and
   a thermal insulation layer is provided between the flow path and the one or more nanocarbon light sources.
(Clause 4) The infrared analysis chip according to Clause 1 or 2, wherein
   the one or more nanocarbon light sources are provided on a surface of the first substrate opposite to the surface facing the second substrate, and
   the first substrate is transparent to the infrared light.
(Clause 5) The infrared analysis chip according to Clause 1 or 2, further including:
   a third substrate supporting the first substrate, wherein
   the one or more nanocarbon light sources are provided between the first substrate and the third substrate,
   the first substrate is formed of a material having a refractive index higher than that of liquid introduced into the flow path, and
   the reflected infrared light reflected by the liquid or the scattered infrared light scattered by the liquid is detected on a back side of the third substrate.
(Clause 6) The infrared analysis chip according to Clause 5, wherein
   the third substrate is formed of a material that absorbs the infrared light, and has a hole or a space in a region that does not overlap the one or more nanocarbon light sources.
(Clause 7) The infrared analysis chip according to Clause 5 or 6, wherein
   the reflected infrared light or the scattered infrared light is infrared light reflected or scattered by the liquid through an evanescent field generated at an interface between the liquid introduced into the flow path and the first substrate.
(Clause 8) The infrared analysis chip according to any one of Clauses 1 through 4, further including:
   a light-receiving element provided on the second substrate, wherein
   the light-receiving element is configured to detect transmitted infrared light transmitted through liquid introduced into the flow path.
(Clause 9) The infrared analysis chip according to c any one of Clauses 1 through 8, wherein
   the one or more nanocarbon light sources are a plurality of nanocarbon light sources, and
   a plurality of filters configured to transmit light of a wavelength in different infrared ranges to the flow path, the filters being respectively provided between the plurality of nanocarbon light sources and the flow path.
(Clause 10) The infrared analysis chip according to any one of Clauses 1 through 9, wherein
   the one or more nanocarbon light sources are a plurality of nanocarbon light sources,
   the first substrate has a first mirror surface,
   the second substrate has a second mirror surface, and
   a plurality of resonators having different resonator lengths respectively corresponding to the plurality of the nanocarbon light sources are formed between the first mirror surface and the second mirror surface.
(Clause 11) The infrared analysis chip according to any one of Clauses 1 through 10, further including:
   a tube communicating with the flow path; and
   a manipulator for injecting or suctioning liquid into or from the flow path via the tube, wherein
   the manipulator propels the liquid introduced into the flow path in a one-dimensional direction or a two-dimensional direction.
(Clause 12) The infrared analysis chip according to any one of Clauses 1 through 11, wherein
   each of the one or more nanocarbon light sources is linear with a line width of 1 µm or greater and 100 µm or less.
(Clause 13) An infrared analysis chip, including:
   a first substrate;
   a second substrate facing the first substrate;
   a spacer separating the first substrate and the second substrate at a predetermined interval; and
   a flow path formed by the spacer between the first substrate and the second substrate, wherein
   at least one of the first substrate or the second substrate is transparent to light of an infrared range, and
   the spacer has a structure in which an adhesive layer and a spacer member contained in the adhesive layer are integrated.
(Clause 14) The infrared analysis chip according to Clause 13, wherein
   the spacer member is particles or projections formed on the first substrate or the second substrate.
(Clause 15) An infrared analysis apparatus, including:
   a substrate; and
   a nanocarbon light source provided on the substrate and configured to irradiate an object to be measured with infrared light by black body radiation, wherein
   the nanocarbon light source emits the infrared light modulated in such a manner that a second temperature difference becomes 0.2 times or less of a first temperature difference, the first temperature difference being a temperature difference in the substrate between when a voltage or current of a first value is continuously applied to the nanocarbon light source and when a voltage or current of a second value is continuously applied to the nanocarbon light source, the second temperature difference being a temperature difference between a maximum temperature of the substrate and a minimum temperature of the substrate when the voltage or the current is modulated in such a manner that the voltage or current of the first value is applied as a maximum value and the voltage or current of the second value is applied as a minimum value.
(Clause 16) An infrared analysis chip, including:
   a flow path into which liquid is introduced; and
   a nanocarbon heater provided in the flow path, wherein
   the nanocarbon heater is provided in the flow path and configured to locally heat the liquid by applying a voltage to generate bubbles, and the bubbles cause the liquid to move within the flow path.
(Clause 17) The infrared analysis chip according to Clause 16, further including:
   a nanocarbon light source for irradiating the flow path with infrared light.
(Clause 18) The infrared analysis chip according to Clause 17, wherein
   the nanocarbon heater propels or transfers the liquid by the bubbles to a region in the flow path that is irradiated by the nanocarbon light source with infrared light.
(Clause 19) The infrared analysis chip according to any one of Clauses 16 through 18, wherein
   the nanocarbon heater is linear with a line width of 1 µm or greater and 100 µm or less.
(Clause 20) A wearable device having an infrared analysis chip, the infrared analysis chip including:
   a first substrate having a surface configured to be in contact with a living body;
   a third substrate configured to support the first substrate; and
   a nanocarbon light source provided between the first substrate and the third substrate and configured to irradiate the living body with infrared light, wherein
   the first substrate is formed of a material having a refractive index higher than that of the living body, and
   reflected infrared light reflected by the living body or scattered infrared light scattered by the living body is detected on a back side of the third substrate.
(Clause 21) An infrared analysis chip, including:
   a flow path;
   a plurality of nanocarbon light sources configured to irradiate the flow path with infrared light; and
   a light-receiving element configured to receive infrared light transmitted through, reflected by, or scattered by the flow path, wherein
   the plurality of nanocarbon light sources are different in size, emission intensity of the infrared light, wavelength of the infrared light, modulation rate of the infrared light, modulation frequency of the infrared light, or emission timing.
(Clause 22) An infrared analysis apparatus, including:
   the infrared analysis chip according to Clause 21;
   a modulator configured to modulate the plurality of nanocarbon light sources at mutually different frequencies; and
   a measuring instrument configured to measure an output signal of the light-receiving element in synchronization with the different frequencies.

This application claims priority to Japanese Patent Application No. 2023-081419, filed May 17, 2023, with the Japan Patent Office, the entire contents of which are hereby incorporated by reference.

### REFERENCE SIGNS LIST

5: Liquid
10, 10A, 10B, 10C, 20, 20A, 20B, 20C, 20D, 30, 30A, 40, 50, 60, 60A, 60B, 60C, 70, 70A, 200: Infrared analysis chip
11, 21, 41, 51, 61, 71: First substrate
12, 22, 42, 52, 62, 72: Second substrate
13, 23, 43, 53, 63, 63A, 63B, 63C, 73: Flow path
14, 44: Adhesive layer
15: Spacer member
15A: Particles
15B, 45: Projections
16, 16-1, 16-2, 26, 26-1, 26-2, 46-1, 46-2, 56, 66, 76: Spacer
24a, 24b: Electrode
25, 25-1, 25-2, 25-3: Nanocarbon light source
28: Thermal insulating layer
31: Third substrate
31A: Hole or space
33a, 33b, 33c: Filter
35: Light-receiving element
47-1, 47-2, 47-3: Resonator
57: Tube
65: Nanocarbon heater
69: Bubbles
80: Wearable device
86, 86A, 86B, 86C: Modulator
88: Measurement instrument
91: First mirror
92, 92-1, 92-2, 92-3: Second mirror
H: Temperature increase region
L1, L2, L3: Resonator length

## Claims

1. An infrared analysis chip, comprising:
a first substrate;
a second substrate facing the first substrate;
a flow path provided between the first substrate and the second substrate; and
one or more nanocarbon light sources thermally insulated from the flow path and configured to irradiate the flow path with infrared light.

2. The infrared analysis chip according to claim 1, wherein
the one or more nanocarbon light sources are a plurality of nanocarbon light sources, and
the plurality of nanocarbon light sources are different in size, emission intensity of the infrared light, wavelength of the infrared light, modulation rate of the infrared light, modulation frequency of the infrared light, or emission timing.

3. The infrared analysis chip according to claim 1, wherein
the one or more nanocarbon light sources are provided on a surface of the first substrate that faces the second substrate, and
a thermal insulation layer is provided between the flow path and the one or more nanocarbon light sources.

4. The infrared analysis chip according to claim 1, wherein
the one or more nanocarbon light sources are provided on a surface of the first substrate opposite to the surface facing the second substrate, and
the first substrate is transparent to the infrared light.

5. The infrared analysis chip according to claim 1, further comprising
a third substrate supporting the first substrate, wherein
the one or more nanocarbon light sources are provided between the first substrate and the third substrate,
the first substrate is formed of a material having a refractive index higher than that of liquid introduced into the flow path, and
the reflected infrared light reflected by the liquid or the scattered infrared light scattered by the liquid is detected on a back side of the third substrate.

6. The infrared analysis chip according to claim 5, wherein
the third substrate is formed of a material that absorbs the infrared light, and has a hole or a space in a region that does not overlap the one or more nanocarbon light sources.

7. The infrared analysis chip according to claim 5, wherein
the reflected infrared light or the scattered infrared light is infrared light reflected or scattered by the liquid through an evanescent field generated at an interface between the liquid introduced into the flow path and the first substrate.

8. The infrared analysis chip according to claim 1, further comprising:
a light-receiving element provided on the second substrate, wherein
the light-receiving element is configured to detect transmitted infrared light transmitted through liquid introduced into the flow path.

9. The infrared analysis chip according to claim 1, wherein
the one or more nanocarbon light sources are a plurality of nanocarbon light sources, and
a plurality of filters respectively configured to transmit light of a wavelength in different infrared ranges to the flow path, the filters being respectively provided between the plurality of nanocarbon light sources and the flow path.

10. The infrared analysis chip according to claim 1, wherein
the one or more nanocarbon light sources are a plurality of nanocarbon light sources,
the first substrate has a first mirror surface,
the second substrate has a second mirror surface, and
a plurality of resonators having different resonator lengths respectively corresponding to the plurality of the nanocarbon light sources are formed between the first mirror surface and the second mirror surface.

11. The infrared analysis chip according to claim 1, further comprising:
a tube communicating with the flow path; and
a manipulator for injecting or suctioning liquid into or from the flow path via the tube, wherein
the manipulator propels the liquid introduced into the flow path in a one-dimensional direction or a two-dimensional direction.

12. The infrared analysis chip according to claim 1, wherein
each of the one or more nanocarbon light sources is linear with a line width of 1 µm or greater and 100 µm or less.

13. An infrared analysis chip, comprising:
a first substrate;
a second substrate facing the first substrate;
a spacer separating the first substrate and the second substrate at a predetermined interval; and
a flow path formed by the spacer between the first substrate and the second substrate, wherein
at least one of the first substrate or the second substrate is transparent to light of an infrared range, and
the spacer has a structure in which an adhesive layer and a spacer member contained in the adhesive layer are integrated.

14. The infrared analysis chip according to claim 13, wherein
the spacer member is particles or projections formed on the first substrate or the second substrate.

15. An infrared analysis apparatus, comprising:
a substrate; and
a nanocarbon light source provided on the substrate and configured to irradiate an object to be measured with infrared light by black body radiation, wherein
the nanocarbon light source emits the infrared light modulated in such a manner that a second temperature difference becomes 0.2 times or less of a first temperature difference, the first temperature difference being a temperature difference in the substrate between when a voltage or current of a first value is continuously applied to the nanocarbon light source and when a voltage or current of a second value is continuously applied to the nanocarbon light source, and the second temperature difference being a temperature difference between a maximum temperature of the substrate and a minimum temperature of the substrate when the voltage or the current is modulated in such a manner that the voltage or current of the first value is applied as a maximum value and the voltage or current of the second value is applied as a minimum value.

16. An infrared analysis chip, comprising:
a flow path into which liquid is introduced; and
a nanocarbon heater provided in the flow path, wherein
the nanocarbon heater is provided in the flow path and configured to locally heat the liquid by applying a voltage to generate bubbles, and the bubbles cause the liquid to move within the flow path.

17. The infrared analysis chip according to claim 16, further comprising:
a nanocarbon light source for irradiating the flow path with infrared light.

18. The infrared analysis chip according to claim 17, wherein
the nanocarbon heater propels or transfers the liquid by the bubbles to a region in the flow path that is irradiated by the nanocarbon light source with infrared light.

19. The infrared analysis chip according to claim 16, wherein
the nanocarbon heater is linear with a line width of 1 µm or greater and 100 µm or less.

20. A wearable device having an infrared analysis chip, the infrared analysis chip including:
a first substrate having a surface configured to be in contact with a living body;
a third substrate configured to support the first substrate; and
a nanocarbon light source provided between the first substrate and the third substrate and configured to irradiate the living body with infrared light, wherein
the first substrate is formed of a material having a refractive index higher than that of the living body, and
reflected infrared light reflected by the living body or scattered infrared light scattered by the living body is detected on a back side of the third substrate.

21. An infrared analysis chip, comprising:
a flow path;
a plurality of nanocarbon light sources configured to irradiate the flow path with infrared light; and
a light-receiving element configured to receive infrared light transmitted through, reflected by, or scattered by the flow path, wherein
the plurality of nanocarbon light sources are different in size, emission intensity of the infrared light, wavelength of the infrared light, modulation rate of the infrared light, modulation frequency of the infrared light, or emission timing.

22. An infrared analysis apparatus, comprising:
the infrared analysis chip according to claim 21;
a modulator configured to modulate the plurality of nanocarbon light sources at mutually different frequencies; and
a measuring instrument configured to measure an output signal of the light-receiving element in synchronization with the different frequencies.
